# EUROPEAN PATENT APPLICATION

(11) **EP 4 620 973 A1**
(43) Date of publication of application: **24.09.2025**
(21) Application number: 24177638.4
(22) Date of filing: 23.05.2024
(51) Int. Cl.: C07K 16/18

(54) **ANTIBODIES TARGETING CELL SURFACE DEPOSITED COMPLEMENT PROTEIN C3D AND USE THEREOF**

(30) Priority: 20.03.2024 US 202418610978
(71) Applicant: The United States of America, as represented by the Secretary, Department of Health and Human Services, Bethesda, Maryland 20892-7788 (US); University of Florida Research Foundation, Inc., Gainesville, FL 32611 (US)
(72) Inventor: WIESTNER, Adrian, Bethesda, 20817 (US); RADER, Christoph, Jupiter, 33458 (US); PENG, Haiyong, Jupiter, 33458 (US); BASKAR, Sivasubramanian, Ellicott City, 21042 (US); GAGLIONE, Erika, Germantown, 20874 (US)
(74) Representative: Maiwald GmbH

(57) **Abstract**

Disclosed are anti-C3d antibodies or fragments thereof. Also disclosed are methods of killing cancer cells, methods of preparing anti-C3d antibodies, and pharmaceutical compositions.

## Description

### STATEMENT REGARDING

### FEDERALLY SPONSORED RESEARCH OR DEVELOPMENT

This invention was made with Government support under project number ZIAHL006070-09 by the National Institutes of Health, National Cancer Institute. The Government has certain rights in this invention.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 37,455 Byte Extensible Markup Language (XML) file named "767544.xml," created on February 15, 2024.

### BACKGROUND OF THE INVENTION

Monoclonal antibodies (mAbs) have become a mainstay of therapy for many cancers. The key effector mechanisms of mAbs are induction of cell death through complement-dependent cytotoxicity (CDC), antibody-dependent cellular cytotoxicity (ADCC), antibody-dependent cell-mediated phagocytosis (ADCMP) and in some cases may include induction of apoptosis. The most commonly used monoclonal antibodies are of mouse origin that have been chimerized or humanized to carry human constant regions (typically the human IgG1 isotype), a requirement for the recruitment of human effector mechanisms.

However, antibody therapy is not completely effective in some applications due to loss of the target surface antigen. For instance, rituximab and ofatumumab are anti-CD20 monoclonal antibodies that mediate human immune effector mechanisms including CDC as well as ADCC and ADCMP and are approved for patients with Chronic Lymphocytic Leukemia (CLL), a B-cell malignancy. Upon infusion of either of these antibodies, complement protein is deposited on the cell surface of CLL cells and a subset of the cells is killed; however, other CLL cells escape, having lost CD20 expression due to a process called trogocytosis by which antibody-CD20 complexes are pulled off the CLL cell surface by immune cells that bind the Fc-portion of the antibody. The process of trogocytosis leading to antigen loss is not limited to anti-CD20 antibodies or leukemia and lymphoma but appears to be a common event in antibody therapy.

While antibodies, such as C8xi, have been developed that successfully overcome problems associated with loss of the targeted antigen (e.g., U.S. Patent No. 10,035,848), there is still a need for new antibody therapies that can overcome problems associated with loss of the targeted antigen.

### BRIEF SUMMARY OF THE INVENTION

Provided is an antibody or antibody fragment immunospecific for human complement protein C3d. According to one aspect of the invention, the anti-C3d antibody or antibody fragment comprises:
(a) a heavy chain immunoglobulin variable region comprising SEQ ID NO: 1, or at least the CDRs thereof; and a light chain immunoglobulin variable region comprising SEQ ID NO: 2, or at least the CDRs thereof;
(b) a heavy chain immunoglobulin variable region comprising SEQ ID NO: 3, or at least the CDRs thereof; and a light chain immunoglobulin variable region comprising SEQ ID NO: 4, or at least the CDRs thereof;
(c) a heavy chain immunoglobulin variable region comprising SEQ ID NO: 5, or at least the CDRs thereof; and a light chain immunoglobulin variable region comprising SEQ ID NO: 6, or at least the CDRs thereof;
(d) a heavy chain immunoglobulin variable region comprising SEQ ID NO: 7, or at least the CDRs thereof; and a light chain immunoglobulin variable region comprising SEQ ID NO: 8, or at least the CDRs thereof; or
(e) a heavy chain immunoglobulin variable region comprising SEQ ID NO: 9, or at least the CDRs thereof; and a light chain immunoglobulin variable region comprising SEQ ID NO: 10, or at least the CDRs thereof.

The invention also provides a method of using the antibody or antibody fragment to kill cells having C3d deposited on their surface.

Related compositions, cells, nucleic acids, and methods also are provided as is apparent from the following detailed description of the invention.

### BRIEF DESCRIPTION OF THE SEVERAL VIEWS OF THE DRAWING(S)

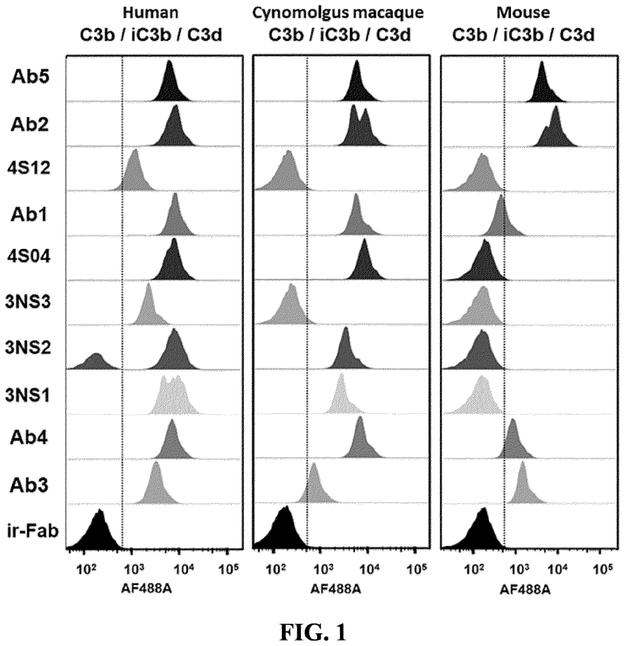

Figure 1 is an image depicting the cross reactivity of anti-C3d Fabs (His-tagged) with human, cynomolgus macaque, or mouse C3b / iC3b / C3d deposited on zymosan particles, as determined by flow cytometry using AF488-conjugated mouse anti-His mAb. Antibodies 4S12, 4S04, 3NS3, 3NS2, and 3NS1 were included for purposes of comparison. A non-binding Fab (irFab) was used as isotype control.

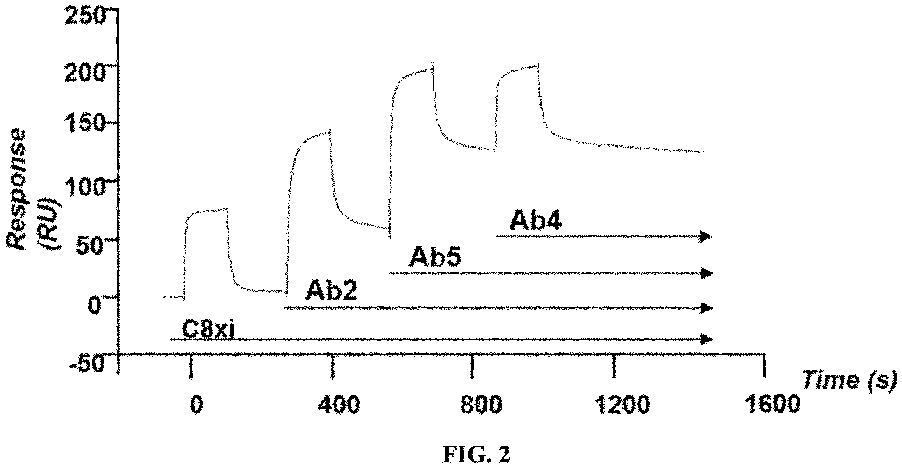

Figure 2 shows a sensorgram showing the binding of anti-C3d Fabs (C8xi, Ab2, Ab5, and Ab4) to the ligand hFc-hC3d that was immobilized on CM5 chip by anti-human Fcy mAb. The Fabs (500 nM) were injected sequentially to identify binding to independent and overlapping epitopes.

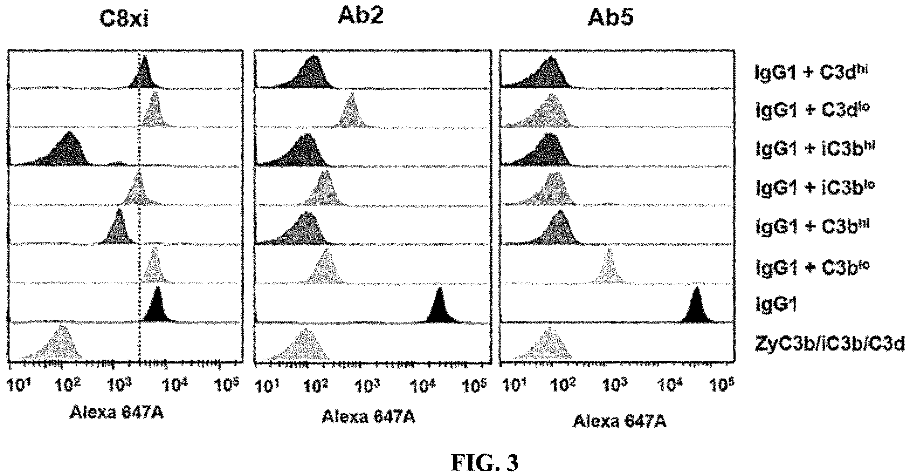

Figure 3 shows an image depicting the results following analysis of the fine specificities of mAbs to C3 fragments by competitive inhibition. Zymosan particles coated with C3 fragments (C3b, iC3b, C3d) for 30 min were incubated with labeled anti-C3d mAbs in the absence or presence of purified soluble C3b, iC3b (10 µg/mL for "lo" and 250 µg/mL for "hi") or C3d (2.5 µg/mL for "lo" or 50 µg/mL for "hi"). The relative specificities for each mAb are: C8xi, iC3b>C3b>C3d; Ab2, iC3b=C3b>C3d; Ab5, C3d> iC3b>C3b.

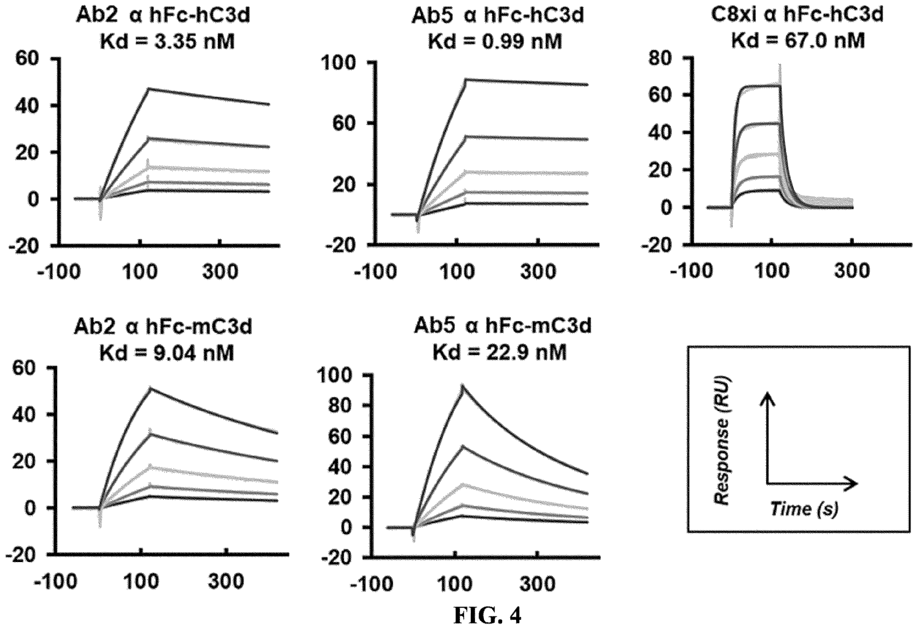

Figure 4 shows a set of sensorgrams following SPR analysis depicting the binding of Ab2, Ab5, and C8xi mAbs at different concentrations (50, 25, 12.5, 6.25, and 3.125 nM) to immobilized hFc-hC3d (top panels) and hFc-mC3d (lower panels).

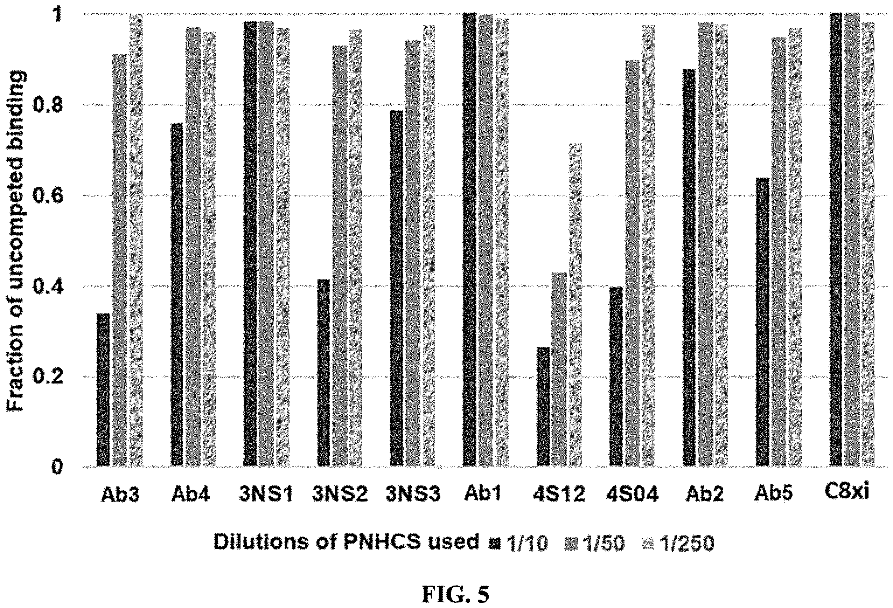

Figure 5 is a graph showing the effect of normal human serum (NHS) on binding of anti-C3d Fabs to human C3d. Zymosan particles opsonized with human C3 fragments were suspended in media with indicated dilutions of NHS (1/10, 1/50 and 1/250) prior to addition of His-tagged anti-C3d Fab antibodies at 1 µg/mL. Mouse anti-His mAb conjugated with HRP was used to determine the binding signal of each Fab by ELISA. Data is normalized to media control containing no NHS. Antibodies 3NS1, 3NS2, 3NS3, 4S12, and 4S04 were included for purposes of comparison.

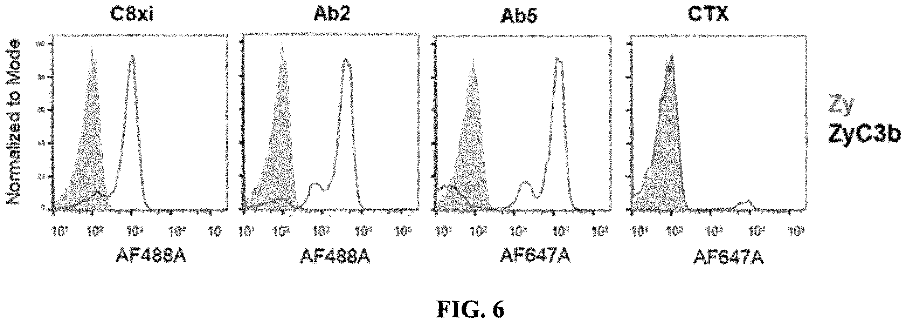

Figure 6 is a set of graphs showing the differential binding of mAbs to "early" C3 fragments (C3b/iC3b) deposited on zymosan particles (ZyC3b) based on incubation for 1 min with NHS. Naive zymosan (Zy) served as a control. Binding of AF488 C8xi, AF647 Ab2, and AF647 Ab5 was determined by flow cytometry. AF647 cetuximab (CTX) was used as a negative control.

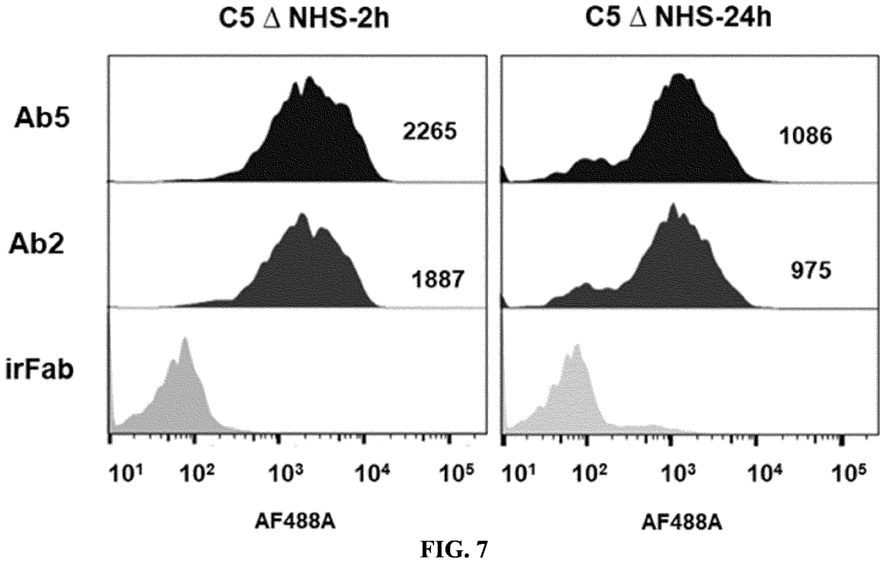

Figure 7 shows the binding of anti-C3d Fabs to JeKo-1 reacted with RTX (Rituximab, an anti-CD20 mAb) in C5△NHS for 2 h and 24 h, to ensure full conversion to C3d. A non-binding Fab (irFab) was used as isotype control, the values depict ΔMFI between control and anti-C3d Fab staining.

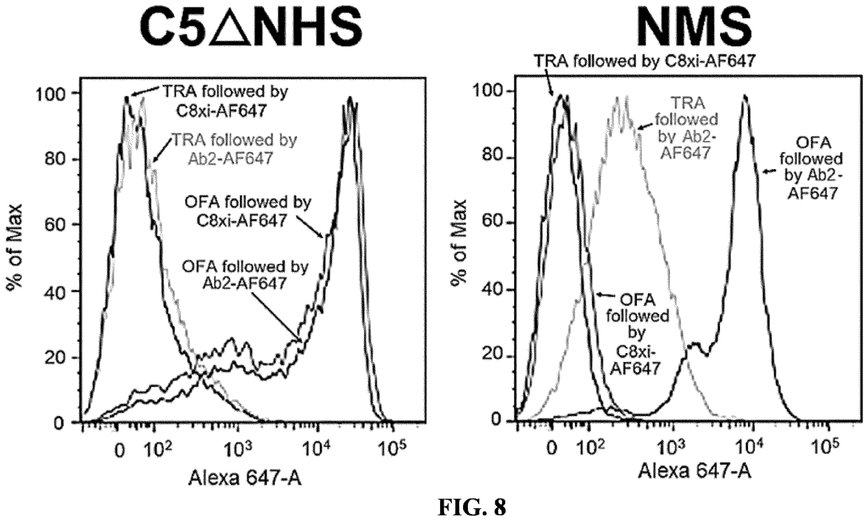

Figure 8 is a set of graphs showing the binding level of mAbs of an aspect of the invention. PBMCs from treatment-naive patients with CLL were reacted with OFA (Ofatumumab, an anti-CD20 mAb) or TRA (Trastuzumab, an anti-HER2 mAb, used as isotype control) in the presence of C5△NHS or normal mouse serum (NMS). After washing, staining with AF647 conjugated mAbs C8xi or Ab2 is shown. As expected, C8xi does not bind to cells opsonized with mouse serum, but rabbit mAb Ab2 binds to cells opsonized with either human or mouse serum.

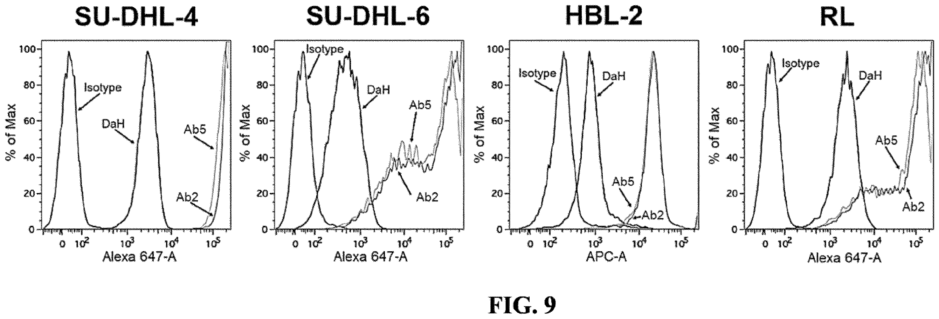

Figure 9 is a set of graphs showing binding level of mAbs of an aspect of the invention. Lymphoma B cell lines were reacted with OFA in the presence of C5△NHS. OFA bound to CD20 on the cell surface was detected using AF647 DaH and bound C3d was detected using AF647 conjugated Ab2 or Ab5 mAbs. Normal human IgG conjugated to AF647 (AF647 HlgG) was used as an isotype control.

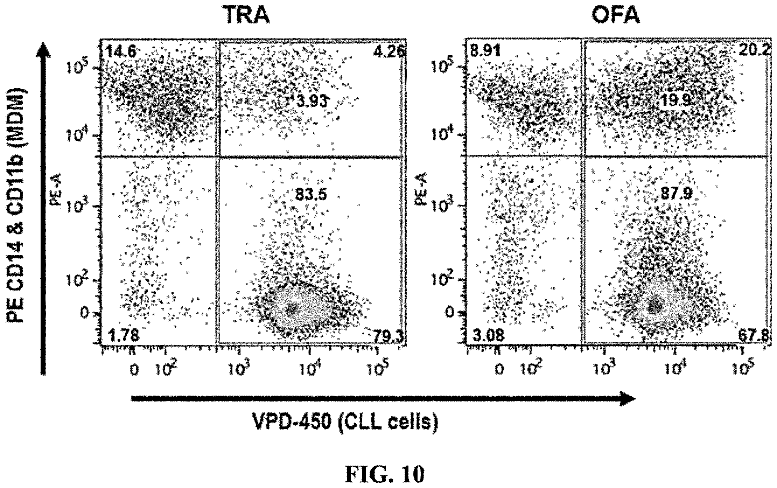

Figure 10 is a set of flow cytometry plots showing ADCP of CLL PBMC as determined by flow cytometry. PBMC obtained from treatment naive patients with CLL were VPD450-labelled (target CLL cells) and opsonized with 2 ug/mL of TRA or OFA. The target cells were co-cultured with human MDM (effector cells) for 2-3 h at 37 °C. After culturing, the cells were labelled with PE-CD11b and PE-CD14 and analyzed by flow cytometry. Double positive events (VPD-450+ and PE+) represent CLL cells phagocytosed by human MDM.

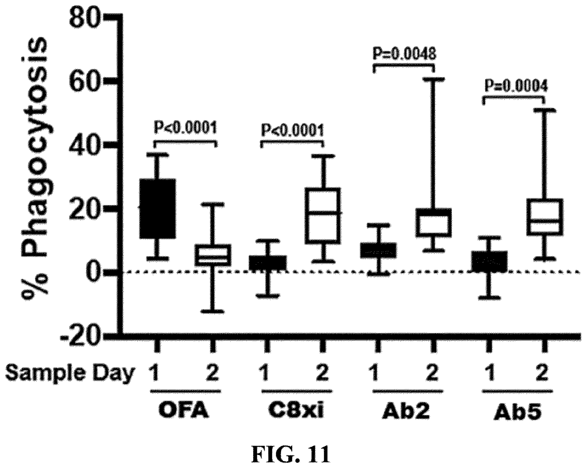

Figure 11 shows ADCP of CLL PBMC as determined by flow cytometry. In vitro phagocytosis of PBMC (based on flow cytometry as in Figure 10) obtained from patients at baseline (Day 1, filled boxes) and one day after treatment with OFA (Day 2, open boxes) are shown. Values depict mean ± SEM of 8-13 samples. The degree of phagocytosis mediated by ofatumumab and anti-C3d mAbs, at their respective time points, was comparable.

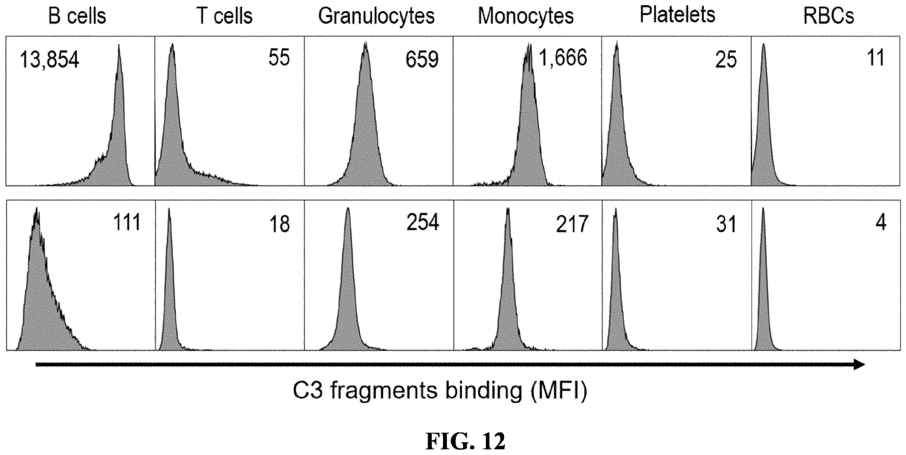

Figure 12 is set of graphs showing the results of a whole blood assay. Blood was obtained from a treatment-naive CLL patient and was washed thoroughly and was then reacted in vitro with Ofatumumab [10 ug/mL] in the presence (upper panel) or absence (lower panel) of C5△NHS. Staining was accomplished using AF647 mAbs (Ab2 or Ab5) along with a panel of fluorochrome conjugated mAbs against phenotype markers to identify individual cell populations. Values in each panel depict MFI of C3 fragment binding to indicated cell populations using Ab2.

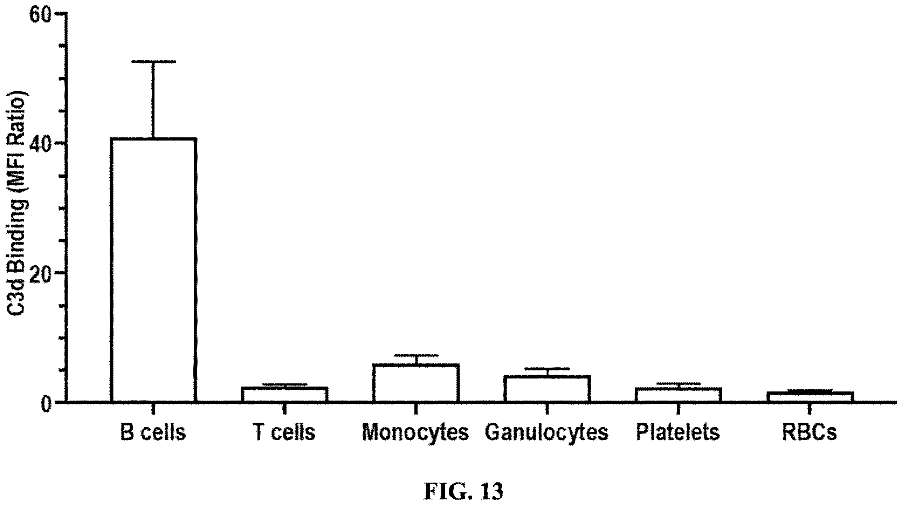

Figure 13 is a graph showing a summary of the data on C3d opsonization of cell populations from 5 patients with CLL. The MFI ratio (mean +/- SEM) was calculated as MFI (OFA + C5△NHS) / (OFA alone). C3d MFI on B cells was significantly higher compared to the value for all other cell populations (*P*<0.0001)

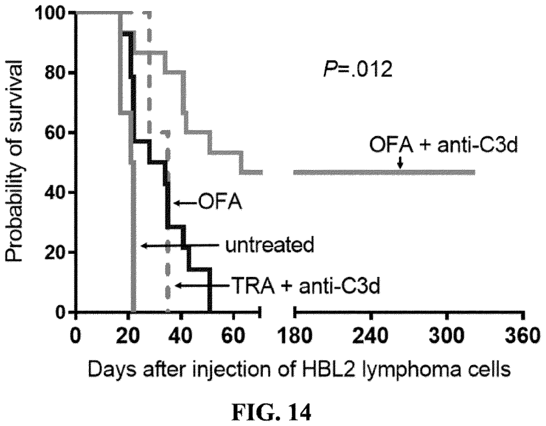

Figure 14 is a graph showing the probability of survival of mice using a mantle-cell lymphoma xenograft model. Balb/c-SCID mice were challenged s.c. with 5 million HBL-2 cells. Mice were randomized on day 7 to no treatment (untreated, n=5) or four weekly i.p. injections of mAbs: 20 mg/kg OFA alone (OFA, n=9); 10 mg/kg TRA + 10 mg/kg anti-C3d mAb Ab5 (n=5); 10 mg/kg OFA + 10 mg/kg anti-C3d mAb (Ab2, n=7; or Ab5, n=8). Probability of survival for OFA treated vs untreated mice *P*=0.001; for OFA + anti-C3d mAb vs OFA, *P*=0.012; for OFA + antiC3d mAb vs TRA + anti-C3d mAb, *P*=0.006; for OFA + anti-C3d mAb vs untreated *P*=0.0001. P value by log rank test. The experiment was terminated after 300 days.

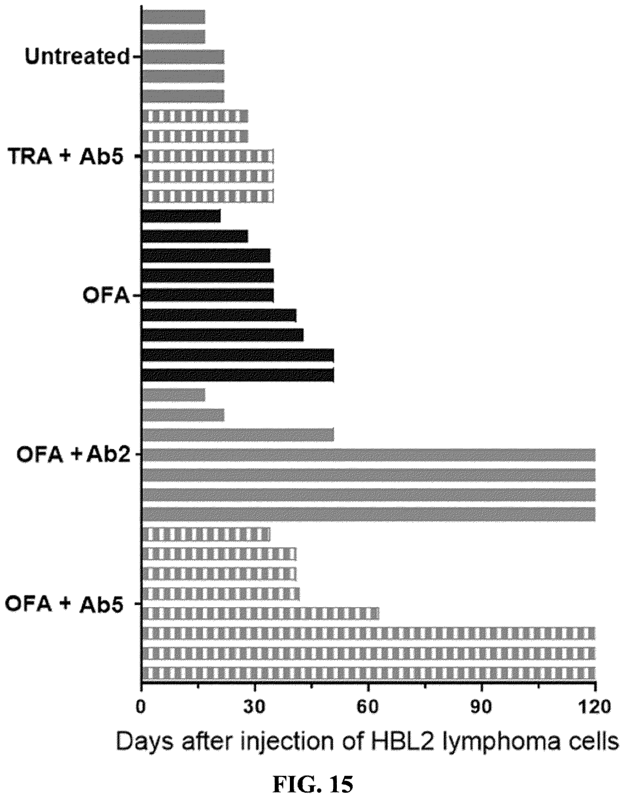

Figure 15 is a graph showing the breakdown of treatment cohorts in the HBL-2 model. Mice were injected with 5 million HBL-2 cells into the flank. Mice were assigned to no treatment or treatment cohorts as indicated (n=4-9 per group). Antibodies were administered i.p. starting one week after tumor challenge and continued for 4 weekly injections. Swimmers' plot indicates survival times to predefined tumor size endpoint; the experiment was terminated 365 days after tumor challenge. The cohorts received either no treatment (No Ab), 20 mg/kg OFA alone or two mAbs combined each at 10 mg/kg: OFA plus Ab2, OFA plus Ab5, or TRA (non-targeting control) plus Ab5.

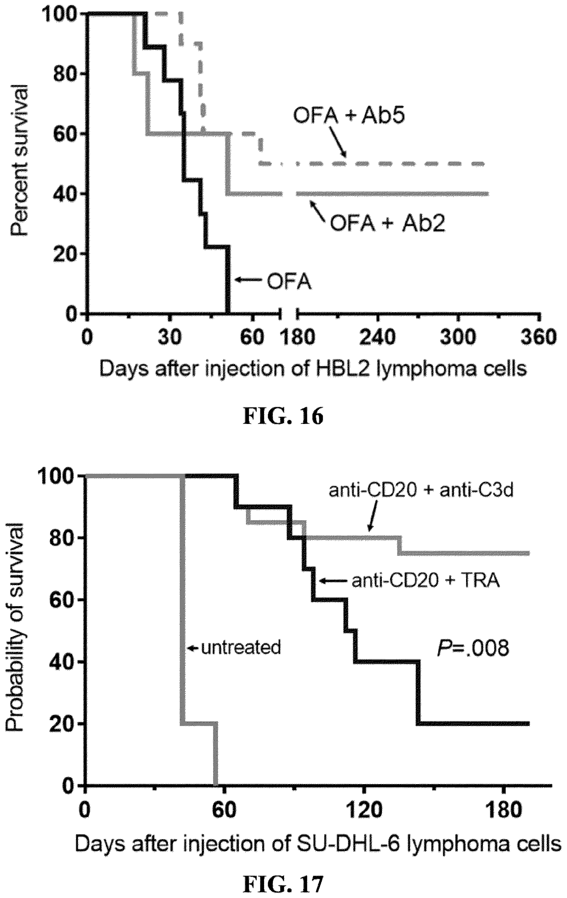

Figure 16 is a graph showing the survival of mice injected with 5 million HBL-2 assigned to treatment cohorts. There was no statistically significant difference based on which anti-C3d mAb was used in the combination therapy.
Figure 17 is a graph showing the probability of survival of mice using a diffuse large B cell lymphoma model. Summarized are data of repeat experiments in Balb/c-SCID mice challenged s.c. with 5 million SU-DHL-6 cells. Approximately half of the mice showed palpable or measurable tumors between day 17 and 28. On day 7, mice were assigned weekly i.p. injections of 10 mg/kg of RTX or OFA combined with 10 mg/kg TRA (n=10) or with an anti-C3d mAb (Ab2 or Ab5; n=20) or left untreated (n=10). A total of eight weekly injections were given. All treatments improved survival (*P*<0.0001). Combination therapy with an anti-C3d and anti-CD20 mAb improved survival over anti-CD20 mAb combined with non-targeting control TRA (*P*=0.008). P value by log rank test. The experiment was terminated on day 191.

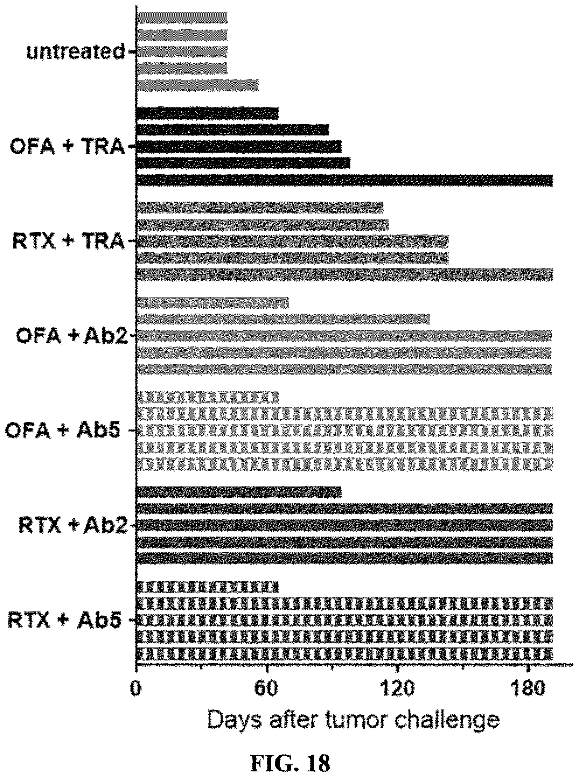

Figure 18 is a graph showing the breakdown of treatment cohorts in the SU-DHL-6 model. Balb/c-SCID mice were challenged s.c. with 5 million SU-DHL-6 cells and randomized to no treatment or indicated treatment groups (n=5 per group). Swimmers' plot indicates survival time or time until termination of the experiment at 6 months from tumor challenge. The cohorts received either no treatment or weekly i.p. injections of 10 mg/kg OFA or 10 mg/kg RTX mixed with 10 mg/kg TRA (non-targeting control), or anti-C3d antibody, either Ab2 or Ab5. A total of eight weekly injections starting on day 7 were given.

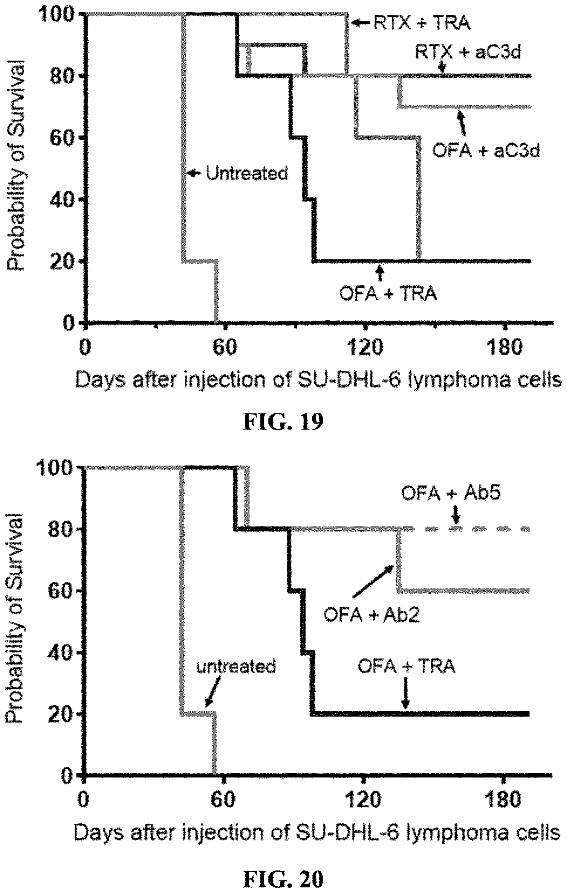

Figure 19 is a graph showing the probability of survival of mice injected with SU-DHL-6 cells assigned to treatment cohorts by type of anti-CD20 mAb used. Median survival for OFA + TRA was 94 days and for RTX + TRA 143 days (*P*=0.21). Survival at day 180 was 80% for RTX + anti-C3d mAb and 70% for OFA + anti-C3d (*P*=0.63).
Figure 20 is a graph showing the probability of survival of mice injected with SU-DHL-6 cells assigned to treatment cohorts by type of anti-C3d mAb used in combination with OFA. There was no statistically significant difference based on which anti-C3d mAb (Ab2 or Ab5) was used in combination with the anti-CD20 mAb OFA.

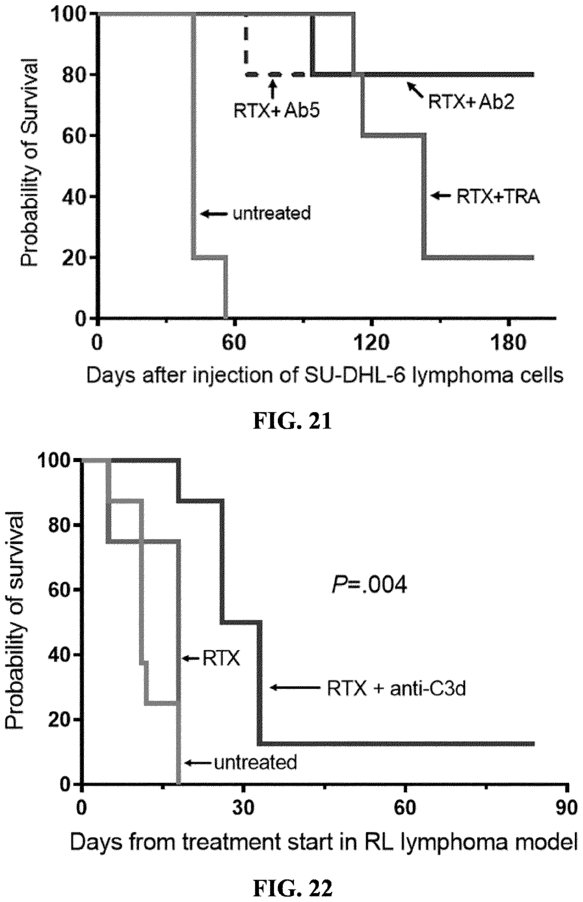

Figure 21 is a graph showing the probability of survival of mice injected with SU-DHL-6 cells assigned to treatment cohorts by type of anti-C3d mAb used in combination with RTX. There was no statistically significant difference based on which anti-C3d mAb (Ab2 or Ab5) was used in combination with the anti-CD20 mAb RTX.
Figure 22 is a graph showing the probability of survival of mice using a follicular lymphoma xenograft model. 10 million RL cells were injected s.c. into the flank of Balb/c-SCID mice. Approximately half of the mice had palpable or measurable tumors on day 21 and all the mice had measurable tumors on day 28 after cell injection. Three weeks after lymphoma challenge, mice were assigned to treatment with 20 mg/kg RTX alone (n=4) or 10mg/kg RTX combined with 10 mg/kg of an anti-C3d mAb (Ab2 (n=4) or Ab5 (n=4) or left untreated (n=8). Weekly mAb injections continued for up to 6 treatments. Survival in the combination arm was significantly improved compared to no treatment (*P*=0.0002) and compared to single agent rituximab (*P*=0.004). RTX alone did not improve survival (*P*=0.21).

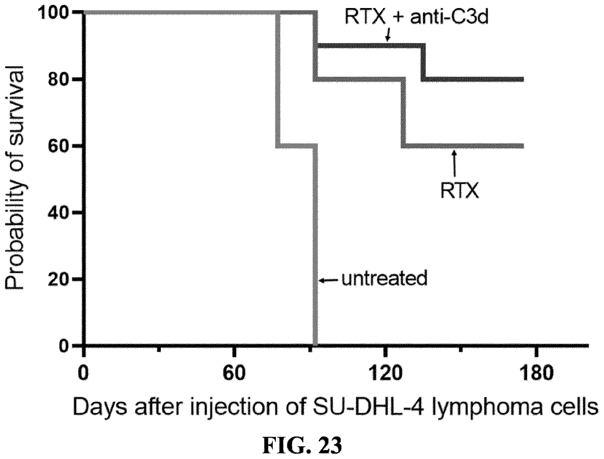

Figure 23 is a graph showing the probability of survival of Balb/c-SCID mice injected s.c. with 20 million SU-DHL-4 cells. On day 7, mice were assigned to receive up to 6 weekly i.p. injections of 20 mg/kg RTX alone (n=5) or 10 mg/kg RTX combined with 10 mg/kg anti-C3d mAb (Ab2 or Ab5, n=10). Survival of mice in both treatment groups was improved compared to untreated controls (*P*=0.013 for RTX alone; *P*=0.0005 for combination therapy). There was no significant difference in survival between treatments (P=0.39). Surviving mice were sacrificed on Day 175 and found to be tumor-free.

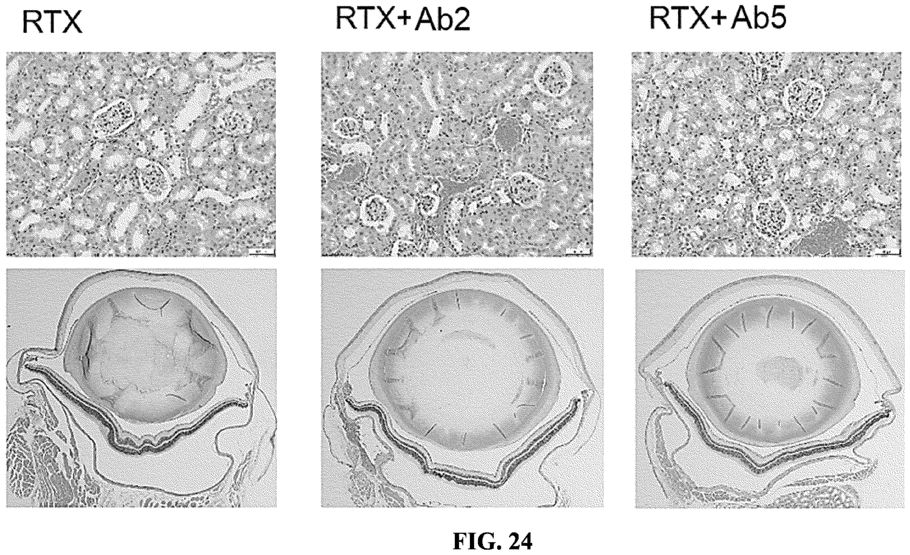

Figure 24 is a set of images of H&E stained kidney and eye tissues. To determine whether the mAbs used in the study caused organ damage, H&E stained slides of kidney, lung, heart, spleen, and eye of 2-3 mice from each treatment group were analyzed on an Olympus BX41 microscope by an expert veterinarian pathologist. No gross changes in morphology were observed in any of the organs analyzed.

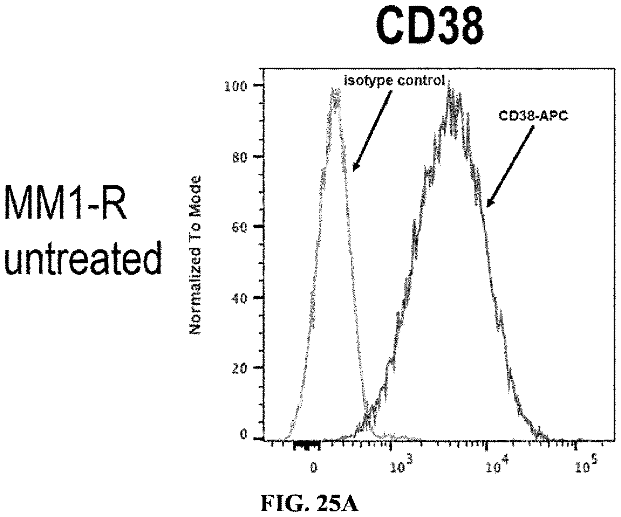

Figure 25A shows CD38 expression on Multiple Myeloma MM1-R cells. Cells were stained with isotype control or CD38-APC at 4 °C.

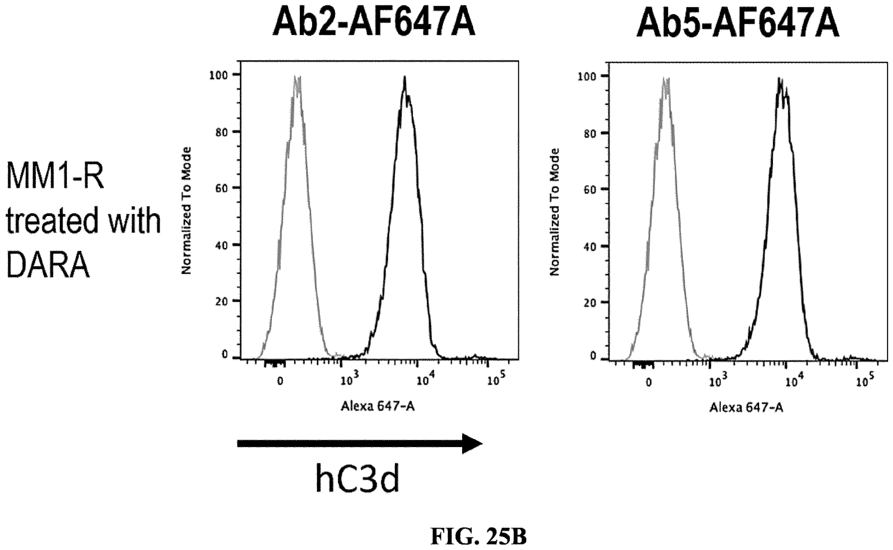

Figure 25B is a set of graphs showing that daratumumab mediates cell surface deposition of hC3d on MM1-R cells *in vitro.* Cells were incubated with 10 ug/mL daratumumab in the presence of C5-depleted human serum (C5△NHS) at 37 °C for 1 h, washed and stained at 4 °C with Ab2-AF647A or Ab5-AF647A to reveal deposited hC3d. The peaks on the left sides of the graphs are isotype control and peaks on the right sides of the graphs are anti-C3d.

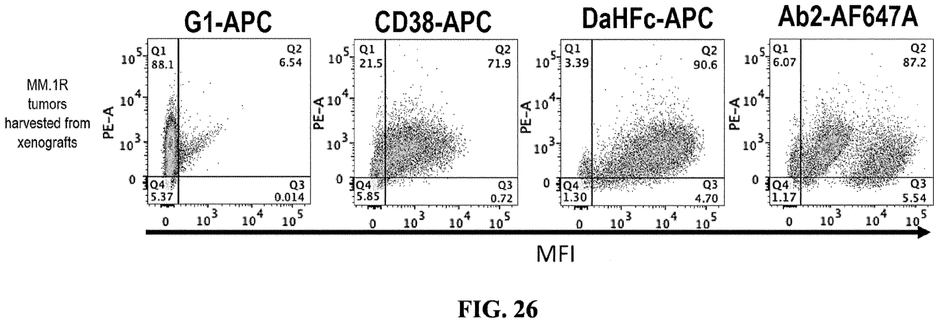

Figure 26 is a set of flow cytometry plots showing *in vivo* deposition of mC3d on MM cells by DARA. Mice bearing MM.1R tumors (given s. c.) received i. p. Dara [10 mg/kg] and tumor cells were isolated the next day and stained with the indicated Ab conjugates, isotype control (G1-APC), anti-CD38 APC, DaHFc-APC demonstrating DARA attached to cells, and Ab2-AF647A demonstrating deposited mC3d.

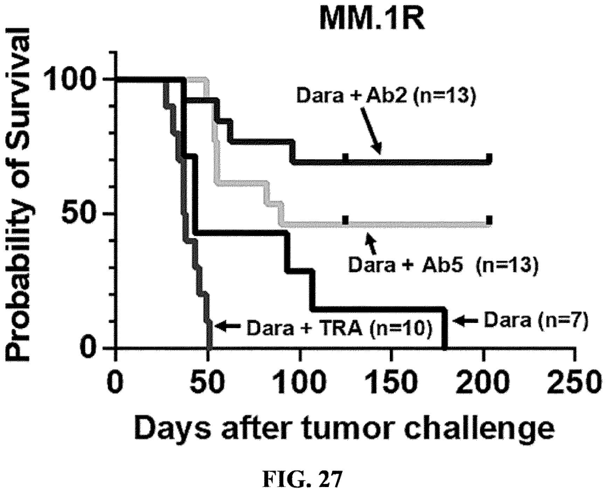

Figure 27 is a graph showing results from a combination treatment of DARA with anti-C3d mAbs of Balb/c-SCID mice bearing MM tumor xenografts. Mice received 1e7 MM.1R tumor cells s.c., tumors were palpable between days 7 and 14. Mice were grouped randomly and starting day 7 received i.p. six weekly followed by two biweekly (total eight) injections of 20 mg/kg DARA alone (n=7), DARA plus TRA, each 10 mg/kg (n=10), DARA plus Ab2, each 10 mg/kg (n=13) or DARA plus Ab5, each 10 mg/kg (n=13). *=P<0.0001

### DETAILED DESCRIPTION OF THE INVENTION

Provided is an antibody or antibody fragment which can enhance efficacy of antibody therapy for cancer. Specifically, provided is an antibody or antibody fragment immunospecific for complement protein C3d, and a method of using the antibody or antibody fragment to kill cells having C3d deposited on the surface thereof. C3d is a protein of the complement system. The complement system consists of soluble plasma proteins and is activated upon binding of a mAb to target cells, resulting in the deposition of complement components on the cell surface and formation of the membrane attack complex (MAC), which can kill cells by forming holes in the cell membrane (lysis). The most abundant complement protein is C3. Upon complement activation, C3 is attracted to the cell surface and activated in a proteolytic step, and the product, activated C3b, is deposited on the cell surface followed later by its proteolytic processing to inactive forms, iC3b, and C3d. C3d is a final product that remains deposited on the cell membrane for days to weeks, while C3b and iC3b are intermediate products that are further processed within hours. C3d, therefore, provides a stable antigenic target. Without wishing to be bound by any particular theory or mechanism of action, it is believed that the antibodies of the invention bind C3d on the surface of a target cell and, thereby, target the cell for destruction by the host's immune system effector cells (e.g., monocytes, macrophages, NK cells, and neutrophils).

The anti-C3d antibody or antibody fragment is immunospecific for human C3d complement protein, particularly human C3d complement protein on the surface of an opsonized cancer cell, and/or the C3d precursor proteins iC3b and C3b that are proteolytically processed to C3d. In some embodiments, the anti-C3d antibody or antibody fragment has a binding affinity (K_{d}) for human C3d protein of at least 500 nM. Desirably, the anti-C3d antibody or antibody fragment has an affinity for C3d that is sufficiently greater than its affinity for other complement proteins that it does not cross react with other complement proteins, particularly C3, which might otherwise compete with C3d for antibody binding, with the exception that the antibody or antibody fragment may cross-react with activated intermediary C3 cleavage products C3b/iC3b.

The anti-C3d antibody or antibody fragment comprises a variable region that contains complementary determining regions (CDRs), which determine the binding specificity of the antibody or antibody fragment. The variable region may include heavy and light chains each comprising CDR regions (wherein the CDRs of the light chain can be referred to as CDRL1, CDRL2, and CDRL3, and the CDRs of the heavy chain can be referred to as CDRH1, CDRH2, and CDRH3. Portions of the variable region flanking and separating the CDR regions are known as framework regions.

An aspect of the invention provides an anti-C3d antibody or antibody fragment comprising, consisting essentially of, or consisting of:
(a) a heavy chain immunoglobulin variable region comprising SEQ ID NO: 1, or at least the CDRs thereof; and a light chain immunoglobulin variable region comprising SEQ ID NO: 2, or at least the CDRs thereof, or sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions;
(b) a heavy chain immunoglobulin variable region comprising SEQ ID NO: 3, or at least the CDRs thereof; and a light chain immunoglobulin variable region comprising SEQ ID NO: 4, or at least the CDRs thereof, or sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions;
(c) a heavy chain immunoglobulin variable region comprising SEQ ID NO: 5, or at least the CDRs thereof; and a light chain immunoglobulin variable region comprising SEQ ID NO: 6, or at least the CDRs thereof, or sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions;
(d) a heavy chain immunoglobulin variable region comprising SEQ ID NO: 7, or at least the CDRs thereof; and a light chain immunoglobulin variable region comprising SEQ ID NO: 8, or at least the CDRs thereof, or sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions; or
(e) a heavy chain immunoglobulin variable region comprising SEQ ID NO: 9, or at least the CDRs thereof; and a light chain immunoglobulin variable region comprising SEQ ID NO: 10, or at least the CDRs thereof, or sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions.

In connection with any of the embodiments provided herein, the CDRs of a given Ig sequence, such as the heavy and light chain sequences mentioned herein, can be determined by any of several conventional numbering schemes, such as Kabat, Chothia, Martin (Enhanced Chothia), IMGT, or AHo (see, e.g., Kabat, et al., Sequences of Proteins of Immunological Interest, U.S. Department of Health and Human Services, NIH (1991); Chothia, et al., J. Mol. Biol., 196: 901-917 (1987); Al-Lazikani et al., J. Mol. Biol., 273: 927-948 (1997); Abhinandan et al., Mol. Immunol., 45: 3832-3839 (2008); Lefranc et al., The Immunologist, 7: 132-136 (1999); Lefranc et al., Dev. Comp. Immunol., 27: 55-77 (2003); and Honegger et al., J. Mol. Biol., 309: 657-670 (2001). In a particular embodiment, the CDRs can be any of those specific CDR sequences provided herein.

An aspect of the invention provides an anti-C3d antibody or antibody fragment comprising, consisting essentially of, or consisting of a heavy chain immunoglobulin variable region and light chain immunoglobulin variable region as set forth Table 1, or at least the CDRs thereof as determined by Kabat, Chothia, Martin (Enhanced Chothia), IMGT, or AHo. In some embodiments, there is provided an anti-C3d antibody or antibody fragment comprising, consisting essentially of, or consisting of a heavy chain immunoglobulin variable region and light chain immunoglobulin variable region as set forth Table 1, or at least the CDRs thereof as determined by Kabat. In some embodiments, there is provided an anti-C3d antibody or antibody fragment comprising, consisting essentially of, or consisting of a heavy chain immunoglobulin variable region and light chain immunoglobulin variable region as set forth Table 1, or at least the CDRs thereof as determined by Chothia. In some embodiments, there is provided an anti-C3d antibody or antibody fragment comprising, consisting essentially of, or consisting of a heavy chain immunoglobulin variable region and light chain immunoglobulin variable region as set forth Table 1, or at least the CDRs thereof as determined by Martin (Enhanced Chothia). In some embodiments, there is provided an anti-C3d antibody or antibody fragment comprising, consisting essentially of, or consisting of a heavy chain immunoglobulin variable region and light chain immunoglobulin variable region as set forth Table 1, or at least the CDRs thereof as determined by IMGT. In some embodiments, there is provided an anti-C3d antibody or antibody fragment comprising, consisting essentially of, or consisting of a heavy chain immunoglobulin variable region and light chain immunoglobulin variable region as set forth Table 1, or at least the CDRs thereof as determined by AHo.

An aspect of the invention provides an anti-C3d antibody or antibody fragment comprising, consisting essentially of, or consisting of a heavy chain immunoglobulin variable region and light chain immunoglobulin variable region as set forth Table 1 (Example 1), or a heavy chain immunoglobulin variable region and light chain immunoglobulin variable region having amino acid sequences with at least about 90%, at least about 91%, at least about 92%, at least about 93%, at least about 94%, at least about 95%, at least about 96%, at least about 97%, at least about 98%, or at least about 99% sequence identity to such heavy and light chain variable regions set forth in Table 1.

An aspect of the invention provides an anti-C3d antibody or antibody fragment comprising, consisting essentially of, or consisting of a heavy chain immunoglobulin variable region and light chain immunoglobulin variable region as set forth Table 1.

An aspect of the invention provides an anti-C3d antibody or antibody fragment comprising, consisting essentially of, or consisting of a heavy chain immunoglobulin variable region and heavy chain immunoglobulin variable region, wherein the heavy chain and light chain variable regions comprise a set of CDRs as set forth in Table 2 (Example 1), as determined by Kabat. Thus, the heavy and light chain variable regions of the anti-C3d antibody or antibody fragment can comprise CDRH1, CDRH2, CDRH2, CDRL1, CDRL2, and CDRL3 corresponding, in order, to:
(a) SEQ ID NOs: 11-16;
(b) SEQ ID NOs: 17-22;
(c) SEQ ID NOs: 23-28;
(d) SEQ ID NOs: 29-34; or
(e) SEQ ID NOs: 35-40;
wherein such CDRs are as determined by Kabat. In some embodiments, the CDRs consist of the sequences set forth above.

Nucleic acid or amino acid sequence "identity," as referenced herein, can be determined by comparing a nucleic acid or amino acid sequence of interest to a reference nucleic acid or amino acid sequence. The percent identity is the number of nucleotides or amino acid residues that are the same (i.e., that are identical) as between the aligned sequence of interest and the reference sequence divided by the length of the longest sequence (i.e., the length of either the sequence of interest or the reference sequence, whichever is longer). A number of mathematical algorithms for obtaining the optimal alignment and calculating identity between two or more sequences are known and incorporated into a number of available software programs. Examples of such programs include CLUSTAL-W, T-Coffee, and ALIGN (for alignment of nucleic acid and amino acid sequences), BLAST programs (e.g., BLAST 2.1, BL2SEQ, BLASTp, BLASTn, and the like) and FASTA programs (e.g., FASTA3x, FASTM, and SSEARCH) (for sequence alignment and sequence similarity searches). Sequence alignment algorithms also are disclosed in, for example, Altschul et al., J. Molecular Biol., 215(3): 403-410 (1990), Beigert et al., Proc. Natl. Acad. Sci. USA, 106(10): 3770-3775 (2009), Durbin et al., eds., Biological Sequence Analysis: Probalistic Models of Proteins and Nucleic Acids, Cambridge University Press, Cambridge, UK (2009), Soding, Bioinformatics, 21(7): 951-960 (2005), Altschul et al., Nucleic Acids Res., 25(17): 3389-3402 (1997), and Gusfield, Algorithms on Strings, Trees and Sequences, Cambridge University Press, Cambridge UK (1997)). Percent (%) identity of sequences can be also calculated, for example, as 100 x [(identical positions)/min(TG_{A}, TG_{B})], where TG_{A} and TG_{B} are the sum of the number of residues and internal gap positions in peptide sequences A and B in the alignment that minimizes TG_{A} and TG_{B}. See, e.g., Russell et al., J. Mol. Biol., 244: 332-350 (1994).

The antibody can be a complete (full) antibody, or an antigen binding antibody fragment. The antibody may be of any immunoglobulin type (e.g., IgG, IgE, IgM, IgD, or IgA), or class (e.g., IgG1, IgG2, IgG3, or IgG4). The antigen binding fragment can be any part of an antibody that has at least one antigen binding site, including, but not limited to, IgGΔCH₂, Fab, F(ab')2, Fv, dsFv, scFv, scFv2CH3, scFv4, scFv3, scFv2, scFv-Fc, diabodies, triabodies, bis-scFvs, (scFv)2, fragments expressed by a Fab expression library, domain antibodies, VHH domains, V-NAR domains, VH domains, VL domains, and the like. The antibody or antibody fragment can be engineered to have various configurations known in the art. For example, the antibody or antibody fragment can be linked to a synthetic molecule with the following domains: a spacer or hinge region (e.g., a CD28, CD28, or IgG hinge), a transmembrane region (e.g., a transmembrane canonical domain), and/or an intracellular T-cell receptor (TCR) signaling domain, thereby forming a T-body or chimeric antigen receptor (CAR). Intracellular TCR signaling domains that can be included in a T-body (or CAR) include, but are not limited to, CD3ζ, FcR-γ, and Syk-PTK signaling domains as well as the CD28, 4-1BB, and CD134 co-signaling domains. Methods for constructing T-cells expressing a T-body (or CAR) are known in the art. See, e.g., Marcu-Malina et al., Expert Opinion on Biological Therapy, 9: 539-564 (2009).

The antibody or antibody fragment includes antibodies that have been mutated or otherwise modified. For instance, the antibody or antibody fragment can comprise a mutation of the Fc-region of a human IgGI heavy chain to enhance effector function, as described in WO 2013/004842. Or, the antibody can be glycoengineered, for instance, to enhance monocyte/macrophage-mediated phagocytosis and cytotoxicity (see, e.g., Herter et al., J. Immunol., 192(5): 2252-60 (2014). The antibody or antibody fragments described herein can be modified in any of various other ways known in the art without departing from the scope of the invention.

A domain antibody comprises a functional binding unit of an antibody, and can correspond to the variable regions of either the heavy (VH) or light (VL) chains of antibodies. A domain antibody can have a molecular weight of approximately 13 kDa, or approximately one-tenth of a full antibody. Domain antibodies may be derived from full antibodies such as those described herein.

The antigen binding fragments in some embodiments are monomeric or polymeric, bispecific or trispecific, bivalent or trivalent. Antibody fragments that contain the antigen binding, or idiotype, of the antibody molecule may be generated by techniques known in the art. For example, such fragments include, but are not limited to, the F(ab')2 fragment which may be produced by pepsin digestion of the antibody molecule; the Fab' fragments which may be generated by reducing the disulfide bridges of the F(ab')2 fragment, and the two Fab' fragments which may be generated by treating the antibody molecule with papain and a reducing agent.

A single-chain variable region fragment (scFv) antibody fragment, which consists of a truncated Fab fragment comprising the variable (V) domain of an antibody heavy chain linked to a V domain of a light antibody chain via a synthetic peptide, can be generated using routine recombinant DNA technology techniques (see, e.g., Janeway et al. (eds.), Immunobiology, 5th Ed., Garland Publishing, New York, NY (2001)). Similarly, disulfide -stabilized variable region fragments (dsFv) can be prepared by recombinant DNA technology (see, e.g., Reiter et al., Protein Engineering, 7: 697-704 (1994)).

Recombinant antibody fragments, e.g., scFvs, can also be engineered to assemble into stable multimeric oligomers of high binding avidity and specificity to different target antigens. Such diabodies (dimers), triabodies (trimers) or tetrabodies (tetramers) are well known in the art, see e.g., Kortt et al., Biomol. Eng., 18: 95-108, (2001) and Todorovska et al., J. Immunol. Methods, 248: 47-66 (2001).

Bispecific antibodies (bscAb) are molecules comprising two single-chain Fv fragments joined via a glycine- serine linker using recombinant methods. The V light-chain (VL) and V heavy-chain (VH) domains of two antibodies of interest in exemplary embodiments are isolated using standard PCR methods. The VL and VH cDNAs are then joined to form a single-chain fragment in a two-step fusion PCR. Bispecific fusion proteins are prepared in a similar manner. Bispecific single-chain antibodies and bispecific fusion proteins are antibody substances included within the scope of the present invention. Exemplary bispecific antibodies are taught in U.S. Patent Application Publication No. 2005-0282233A1 and International Patent Application Publication No. WO 2005/087812, both applications of which are incorporated herein by reference in their entirety. The multispecific antibody can be configured as a BiTE or DART. BiTEs consist of a single polypeptide displaying two antigen-binding specificities through cognate heavy and light chain variable domains. BiTEs have one N-terminus and one C-terminus. In DARTs, cognate heavy and light chain variable domains are on two separate polypeptides that associate and are stabilized by a C-terminal disulfide bridge. Thus, DARTs have 2 N-termini and 2 C-termini.

The anti-C3d antibody can be made by any suitable technique. The antibody is an engineered antibody produced by synthetic, recombinant, or other manufacturing techniques. Suitable methods of making engineered antibodies are known in the art. For instance, a polyclonal antibody can be prepared by immunizing an animal with an immunogen (e.g., C3d) and collecting antisera from that immunized animal. A wide range of animal species can be used for the production of antisera. In some aspects, an animal used for production of antisera is a non-human animal including rabbits, mice, rats, hamsters, goat, sheep, pigs or horses. Polyclonal antisera may be obtained, after allowing time for antibody generation, simply by bleeding the animal and preparing serum samples from the whole blood. The polyclonal antibodies, thus, obtained can then be screened for specific desired antibodies (e.g., antibodies of the invention).

Monoclonal antibodies may be prepared using any technique which provides for the production of antibody molecules by continuous cell lines in culture. Several hybridoma methods are known in the art (e.g., Koehler and Milstein, Nature, 256: 495-497 (1975); Kosbor et al., Immunol Today, 4: 72 (1983); Cote et al., Proc. Natl. Acad. Sci., 80: 2026-2030, 1983); Cole et al., Monoclonal Antibodies and Cancer Therapy, Alan R Liss Inc, New York N.Y., pp 77-96, (1985); Harlow and Lane (eds.), Antibodies: A Laboratory Manual, CSH Press (1988), and CA. Janeway et al. (eds.), Immunobiology, 5th Ed., Garland Publishing, New York, NY (2001); Haskard and Archer, J. Immunol. Methods, 74(2): 361-67 (1984); Roder et al., Methods Enzymol., 121: 140-67 (1986); Huse et al., Science, 246: 1275-81 (1989)). Other known antibody production techniques can also be used, such as by producing human antibodies in non-human animals (e.g., U.S. Patents 5,545,806, 5,569,825, and 5,714,352, and U.S. Patent Application Publication No. 2002/0197266), screening methods (e.g., Orlandi et al., Proc. Natl. Acad. Sci., 86: 3833-3837 (1989), and Winter et al., Nature, 349: 293-299 (1991); phage display methods (e.g., Sambrook et al. (eds.), Molecular Cloning, A Laboratory Manual, 3 Edition, Cold Spring Harbor Laboratory Press, New York (2001); use of transgenic mice (e.g., U.S. Patents 5,545,806 and 5,569,825).

Methods for generating engineered and humanized antibodies are well known in the art (e.g., Janeway et al. (eds.), Immunobiology, 5th Ed., Garland Publishing, New York, NY (2001); U.S. Patents 5,225,539, 5,585,089, 5,693,761, and 5,693,762; European Patent No. 0239400 B1, and United Kingdom Patent No. 2188638; Jones et al., Nature, 321: 522-525 (1986); Riechmann et al., Nature, 332: 323-327 (1988) and Verhoeyen et al., Science 239: 1534-1536 (1988); U.S. Patent 5,639,641; Pedersen et al., J. Mol. Biol., 235: 959-973 (1994); and Owens and Young, J. Immunol. Meth., 168: 149-165 (1994).

Techniques developed for the production of "chimeric antibodies," the splicing of mouse antibody genes to human antibody genes to obtain a molecule with appropriate antigen specificity and biological activity, can be used (Morrison et al., Proc. Natl. Acad. Sci., 81: 6851-6855 (1984); Neuberger et al., Nature, 312: 604-608 (1984); Takeda et al., Nature, 314: 452-454 (1985)). Also, techniques described for the production of single chain antibodies can be employed (U.S. Pat. No. 4,946,778). If a preferred embodiment, the antibodies of the invention are chimeric rabbit/human antibodies.

Chemically constructed bispecific antibodies may be prepared by chemically crosslinking heterologous Fab or F(ab')2 fragments by means of chemicals such as heterobifunctional reagent succinimidyl-3-(2-pyridyldithiol)-propionate (SPDP, Pierce Chemicals, Rockford, 111.). The Fab and F(ab')2 fragments can be obtained from intact antibody by digesting it with papain or pepsin, respectively (Karpovsky et al., J. Exp. Med., 160: 1686-701 (1984); Titus et al., J. Immunol., 138: 4018-22 (1987)).

Methods of testing antibodies for the ability to bind to C3d, regardless of how the antibodies are produced, are known in the art and include any antibody-antigen binding assay, such as, for example, radioimmunoassay (RIA), enzyme-linked immunosorbent assay (ELISA), Western blot, immunoprecipitation, and competitive inhibition assays (see, e.g., Janeway et al. (eds.), Immunobiology, 5th Ed., Garland Publishing, New York, NY (2001); and U.S. Patent Application Publication No. 2002/0197266 A1).

The antibody can be isolated. The term "isolated" as used herein encompasses compounds or compositions that have been removed from a biological environment (e.g., a cell, tissue, culture medium, body fluid, etc.), or otherwise increased in purity to any degree (e.g., isolated from a synthesis medium). Isolated compounds and compositions, thus, can be synthetic or naturally produced.

Also provided is a nucleic acid encoding the anti-C3d antibody as described herein, which can be used to produce the antibody by expressing the nucleic acid in a cell. The nucleic acid can comprise any suitable nucleotide sequence that encodes the antibody or portion thereof (e.g., CDRs, framework regions, and other parts of the antibody or antibody fragment). A nucleic acid comprising the desired nucleotide sequence can be constructed based on chemical synthesis and/or enzymatic ligation reactions using procedures known in the art (e.g., The nucleic acids in some aspects are constructed based on chemical synthesis and/or enzymatic ligation reactions using procedures known in the art. See, for example, et al. (eds.), Molecular Cloning, A Laboratory Manual, 3 Edition, Cold Spring Harbor Laboratory Press, New York (2001).

Also provided is a recombinant expression vector comprising the nucleotide sequence encoding the antibody or antibody fragment. The recombinant expression vector of the invention can be any suitable recombinant expression vector, and can be used to transform or transfect any suitable host. Suitable vectors include those designed for propagation and expansion or for expression or both, such as plasmids and viruses. Examples of vectors include the pUC series (Fermentas Life Sciences), the pBluescript series (Stratagene, La Jolla, CA), the pET series (Novagen, Madison, Wl), the pGEX series (Pharmacia Biotech, Uppsala, Sweden), and the pEX series (Clontech, Palo Alto, CA). Bacteriophage vectors, such as λ 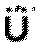 TIO, λ 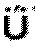 TI 1, AZapll (Stratagene), EMBL4, and λNMI 149, also can be used. Examples of plant expression vectors include pBIOI, pBI101.2, pBI101.3, pBI121 and pBIN19 (Clontech). Examples of animal expression vectors include pEUK-CI, pMAM and pMAMneo (Clontech). The recombinant expression vector can be a viral vector, e.g., a retroviral vector.

Constructs of expression vectors, which are circular or linear, can be prepared to contain a replication system functional in a prokaryotic or eukaryotic host cell. Replication systems can be derived, e.g., from ColEl, 2 µ plasmid, λ, SV40, bovine papilloma virus, and the like.

The recombinant expression vector can comprise regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA- based.

The recombinant expression vector may include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the presently disclosed expression vectors include, for instance, neomycin/G418 resistance genes, hygromycin resistance genes, histidinol resistance genes, tetracycline resistance genes, and ampicillin resistance genes.

The recombinant expression vector can comprise a native or non-native promoter operably linked to the nucleotide sequence encoding the polypeptide (including functional portions and functional variants thereof), or to the nucleotide sequence which is complementary to or which hybridizes to the nucleotide sequence encoding the polypeptide. The selection of promoters, e.g., strong, weak, inducible, tissue-specific and developmental- specific, is within the ordinary skill of the artisan. Similarly, the combining of a nucleotide sequence with a promoter is also within the skill of the artisan. The promoter can be a non- viral promoter or a viral promoter, e.g., a cytomegalovirus (CMV) promoter, an SV40 promoter, an RSV promoter, and a promoter found in the long-terminal repeat of the murine stem cell virus.

The recombinant expression vectors of the invention can be prepared using standard recombinant DNA techniques described in, for example, Sambrook et al., supra.

The nucleic acid or vector can be in a host cell. The host cell can be any type of cell. The host cell in some aspects is a eukaryotic cell, e.g., plant, animal, fungi, or algae, especially a human cell, or can be a prokaryotic cell, e.g., bacteria or protozoa. The host cell in some aspects is a cultured cell or a primary cell, i.e., isolated directly from an organism, e.g., a human. The host cell in some aspects is an adherent cell or a suspended cell, i.e., a cell that grows in suspension. Suitable host cells are known in the art and include, for instance, DH5a E. coli cells, Chinese hamster ovarian cells, monkey VERO cells, COS cells, HEK293 cells, and the like.

Thus, the invention further provides eukaryotic or non-eukaryotic cells that have been recombinantly engineered to produce an antibody or antibody fragment of the invention. The cells can be targeted immune cells that are engineered to recombinantly express the antiC3d antibody or antibody fragment as a cell surface reactive antibody or antibody fragment, such as a T-body or chimeric antigen receptor (CAR). For example, cell can be a T-cell engineered to express an antibody or antibody fragment of the invention (e.g., an scFv, scFv-Fc, or (scFv)2) linked to a spacer or hinge region (e.g., a CD28, CD28, or IgG hinge), a transmembrane region (e.g., a transmembrane canonical domain), and an intracellular T-cell receptor (TCR) signaling domain, thereby forming a T-body or CAR. Intracellular TCR signaling domains that can be included in a T-body (or CAR) include, but are not limited to, CD3ζ, FcR-γ, and Syk-PTK signaling domains as well as the CD28, 4-1BB, and CD134 co-signaling domains. Methods for constructing T-cells expressing a T-body (or CAR) are known in the art. See, e.g., Marcu-Malina et al., Expert Opinion on Biological Therapy, 9: 539-564 (2009).

The anti-C3d antibody or fragment thereof may be conjugated or fused to another molecule, or to a support, optionally by way of a linker molecule. Any of a variety of molecules can be conjugated or fused to the anti-C3d antibody for various purposes, including diagnostic, marking or tracing, therapeutic, or recovery/purification purposes. Examples of such other molecules include, without limitation, detectable labels, affinity tags, and therapeutic agents, including cytotoxic, cytostatic, or antiangiogenic agents and radioisotopes. Therapeutic agents can be, for example, a plant, fungal, or bacterial molecules (e.g., a protein toxin), small molecule chemotherapeutics, or biological therapeutics. Examples of therapeutic molecules include, for instance, a maytansinoid (e.g., maytansinol or DM1 maytansinoid), a taxane, a calicheamicin, an antimetabolite (e.g., an antifolate such as methotrexate, a fluoropyrimidine such as 5-fluorouracil, cytosine arabinoside, or an analogue of purine or adenosine); an intercalating agent (for example, an anthracycline such as doxorubicin, daunomycin, epirubicin, idarubicin, mitomycin-C, dactinomycin, or mithramycin); a platinum derivative (e.g., cisplatin or carboplatin); an alkylating agent (e.g., nitrogen mustard, melphalan, chlorambucil, busulphan, cyclophosphamide, ifosfamide nitrosoureas, or thiotepa); an antimitotic agent (e.g., a vinca alkaloid like vincristine or taxoid such as paclitaxel or docetaxel); a topoisomerase inhibitor (for example, etoposide, and teniposide, amsacrine, or topotecan); a cell cycle inhibitor (for example, a flavopyridol); a microbtubule agent (e.g., an epothilone, discodermolide analog, or eleutherobin analog); a proteosome inhibitor or a topoisomerase inhibitor such as bortezomib, amsacrine, etoposide, etoposide phosphate, teniposide, or doxorubicin; a radioisotope including yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh); an antiangiogenic agent such as linomide, bevacuzimab, angiostatin, and razoxane; an antibody or antibody fragment other than an anti-C3d antibody or antibody fragment, such as rituximab or bevacuzimab. Labels can be useful in diagnostic applications and can include, for example, radiolabels contrast agents. A contrast agent can be a radioisotope label such as iodine (¹³¹I or ¹²⁵I), indium (¹¹¹In), technetium (⁹⁹Tc), phosphorus (³²P), carbon (¹⁴C), tritium (³H), other radioisotope (e.g., a radioactive ion), or a therapeutic radioisotope listed above. Additionally, contrast agents can include radiopaque materials, magnetic resonance imaging (MRI) agents, ultrasound imaging agents, and any other contrast agents suitable for detection by a device that images an animal body; as well as a fluorescent label, a biologically active enzyme label, a luminescent label, or a chromophore label.

Methods of conjugating or fusing such other molecules to an antibody without interfering with the binding of the antibody to its target antigen are known in the art. Recombinant engineering and incorporated selenocysteine (e.g., as described in International Patent Application Publication WO 2008/122039) can be used to conjugate a synthetic molecule. Other methods of conjugation can include covalent coupling to native or engineered lysine side-chain amines or cysteine side-chain thiols. See, e.g., Wu et al., Nat. Biotechnol., 23: 1137-1146 (2005).

The anti-C3d antibody or antibody fragment can be part of a composition, particularly a pharmaceutical composition, comprising the anti-C3d antibody or fragment and a carrier. Any carrier suitable for proteins, particularly antibodies, can be used. A pharmaceutically acceptable carrier is preferred. The term "pharmaceutically acceptable carrier" as used herein means one or more compatible solid or liquid fillers, diluents, other excipients, or encapsulating substances which are suitable for administration into a human or veterinary patient. The carrier can be co-mingled with the one or more active components without substantially impairing the desired pharmaceutical efficacy. Pharmaceutically acceptable materials generally are capable of administration to a patient without the production of significant undesirable physiological effects. The pharmaceutical composition can contain suitable buffering agents, preservatives, and other components typically used in pharmaceutical formulations, particularly therapeutic antibody formulations. The pharmaceutical composition can be presented in a unit dosage form suitable for the desired route of administration (e.g., oral, parenteral, etc).

The anti-C3d antibody can be used for any purpose, such as for labeling opsonized cells, or targeting opsonized cells for delivery of a therapeutic agent. Thus, the invention provides, in one aspect, a method of labeling a cell comprising C3d complement protein on the surface thereof (e.g., an opsonized cell) by contacting the cell with an anti-C3d antibody as described herein that contains a detectable label. According to another aspect, the invention provides a method of delivering a therapeutic agent to a cell comprising C3d complement protein on the surface thereof (e.g., an opsonized cell) by contacting the cell with an anti-C3d antibody as described herein attached to a therapeutic agent. All aspects of the anti-C3d antibody attached to a detectable label or therapeutic agent are as previously described.

The anti-C3d antibody is believed to be particularly useful for eliminating cells, especially cancer cells, by binding to C3d surface proteins on such cells and causing their destruction by cell lysis or phagocytosis. Thus, the invention further provides a method of killing cancer cells comprising C3d complement protein on the surface thereof (e.g., a C3d opsonized, viable cancer cell) by contacting the cell with the anti-C3d antibody described herein, wherein the immune system of the subject is recruited to kill the cancer cell. Alternatively, the method can comprise administering anti-C3d antibody conjugated to a cytotoxic agent to the subject, wherein the anti-C3d antibody targets cancer cells with C3d on the surface and the cytotoxic agent kills the cells. In this respect, killing cancer cells is not limited to direct killing of cancer cells, but includes any method or mechanism by which a living, viable cancer cell may be eliminated from a host as a result of contacting the cancer cell with an antibody of the invention. The invention further provides a method of reducing or eliminating metastasis.

The cell may be any type of cell having a C3d surface protein. Typically, the cell will be a cancer cell or other pathogenic cell having a C3d surface protein (e.g., an opsonized cancer cell or other pathogenic cell). The cancer cell may acquire C3d surface proteins through binding of a mAb to molecules on the surface of the cancer cell. The cancer cell can be a cell of any type of cancer that has a C3d protein on the cell surface. Non-limiting examples of specific types of cancers include cancer of the head and neck, eye, skin, mouth, throat, esophagus, chest, bone, lung, colon, sigmoid, rectum, stomach, prostate, breast, ovaries, uterus (e.g., endometrium), kidney, liver, pancreas, brain, intestine, heart or adrenals. More particularly, cancers include solid tumor, sarcoma, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendothelio sarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma, retinoblastoma, a blood-born tumor, acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acute non-lymphocyctic leukemia, acute undifferentiated leukemia, chronic myelocytic leukemia, chronic lymphocytic leukemia, hairy cell leukemia, or multiple myeloma. See, e.g., Harrison's Principles of Internal Medicine, Eugene Braunwald et al., eds., pp.491-762 (15th ed. 2001).

In an aspect of the invention, the cancer cell is a non-small cell lung cancer cell. In a further aspect, the non-small cell lung cancer is a liver kinase B1 (LKB1)-mutant non-small cell lung cancer. Deng et al. found that in comparison with other genetic subsets, LKB1-mutant NSCLC show marked overexpression of CD38 on the surface of tumor cells (*bioRxiv,* (online April 2023 preprint publication), doi: 10.1101/2023.04.18.537350). Treatment with the FDA-approved anti-CD38 antibody, daratumumab, inhibited growth of LKB1-mutant NSCLC xenografts in mice. Together, these results revealed CD38 as a promising therapeutic target in patients with LKB1 mutant lung cancer.

The methods of the invention are believed to be especially useful to target a cancer cell that has lost a therapeutic target antigen through trogocytosis. In a specific embodiment, the cell is a chronic lymphocytic leukemia cell, and the method may be performed on a patient with chronic lymphocytic leukemia. In another embodiment, the cancer cell is a B-cell expressing CD20, and the method may be performed on a patient with CD20+ B-cell malignancy. In yet another embodiment, the cell is from a cancer treated with a mAb and the method may be performed on a patient in need of treatment for such a cancer.

Any of the foregoing methods may further comprise inducing the formation of C3d on the surface of the cell. This may be accomplished by contacting the cell with an agent that activates the complement system, such as by opsonizing the cell by contact with an antibody or antigen-binding fragment thereof that binds to a surface protein on the cell other than C3d. The antibody that binds to a cell-surface protein other than C3d can be, for example, a therapeutic antibody or antibody fragment. Examples include an anti-CD20 antibody or antibody fragment (e.g., rituximab, ofatumumab, ocrelizumab, veltuzumab, obinutuzumab (GA-101), PRO131921, or ocaratuzumab (AME-133)), an anti-CD33 antibody or antibody fragment (e.g., gemtuzumab, or lintuzumab), an anti-CD38 antibody (e.g., daratumumab), an anti-CD52 antibody (e.g., Alemtuzumab), an anti-ERBB2 antibody (e.g., trastuzumab), and an anti-EGFR antibody (e.g., cetuximab, or panitumumab).

The anti-C3d antibody or antibody fragment and the antibody or antibody fragment that binds to a surface protein on the cell other than C3d can be separate molecules or they can be part of the same multi-specific antibody. Thus, for instance, a multi-specific antibody having immunospecificity for C3d and a different cell-surface protein (e.g., CD20, CD33, CD38, etc.) can be used to both induce the complement cascade resulting in C3d formation on the cell surface and to bind C3d once formed. If separate antibodies or antibody fragments are used to induce C3d formation on the cell surface and bind to C3d once formed, they can be administered to the subject simultaneously or sequentially in any order, though typically the antibody that binds to a cell-surface protein other than C3d will be administered before or approximately at the same time as the anti-C3d antibody.

In accordance with any of the foregoing methods, the cell may be in vitro or in vivo. For instance, the cell may be in a patient, who may be afflicted with a disease. When the cell is in the patient, the cell may be contacted by the anti-C3d antibody or other agent by administering the antibody or other agent to the patient. If the patient is afflicted with a disease, the administration of the anti-C3d antibody and/or other agents can, according to certain embodiments, treat the disease by reducing one or more symptoms or characteristics of the disease. The patient can be a patient treated with a therapy (e.g., monoclonal antibody therapy, particularly anti-CD20 monoclonal antibody therapy, such as with rituximab or ofatumumab) resulting in C3d deposition on the cancer cells. In one embodiment trogocytosis may lead to loss of the CD20 antigen and anti-C3d mAb can be used to target these cells. In a second embodiment anti CD20 therapy leads to C3d deposition but incomplete killing of the cancer cell, that can then be eliminated by anti-C3d mAb alone, or by the combination of the anti CD20 mAb with the anti-C3d mAb.. In one embodiment anti-CD20 mAb is combined with anti-C3d mAb for the treatment of CD20 positive B-cell malignancies.

Thus, for instance, the method may comprise administering to a patient, simultaneously or sequentially, an anti-C3d antibody as described herein and an agent that induces formation of C3d on the surface of a cell (e.g., a monoclonal antibody, such as an anti-CD20 antibody like rituximab, ofatumumab, ocrelizumab, veltuzumab, obinutuzumab (GA-101), PRO131921, ocaratuzumab (AME-133) or any antibody or antibody fragment to a cell surface antigen described herein, or other opsonizing agent). The cell is particularly a pathogenic cell such as a cancer cell. By way of further example, the cell can be a chronic lymphocytic leukemia cell, and the patient can have chronic lymphocytic leukemia.

Further, the method may comprise administering to a patient, simultaneously or sequentially, an anti-C3d antibody as described herein and an anti-CD38 antibody such as daratumumab, or a fragment thereof. The cell targeted by the treatment is particularly a pathogenic cell such as a cancer cell. By way of further example, the cell targeted by the treatment can be a plasma cell, and the patient can have multiple myeloma.

The anti-C3d antibody and agent that induces formation of C3d on the surface of a cell can be administered simultaneously as separate compositions or as a single composition, or the two agents can be administered sequentially in any order. When administered sequentially, the timeframe of administration is not particularly limited, but the anti-C3d antibody and agent that induces C3d formation on the cell will typically be administered within several minutes (e.g., within 10, 20, 30, 40, or 50 minutes) or within several hours (e.g., within 1, 2, 4, 8, 12, or 24 hours), or within several days (e.g., 1, 2, 5, 7), or within several weeks (e.g., 1, 2, 3, 4) of one another.

The polypeptide can be bound to a support, directly or via a linker molecule. The support can be any type of support (e.g., solid supports, such as a bead or plate), particular a support useful in biopanning techniques, such as panning a phage display library.

The polypeptide can be used for any suitable purpose. For instance, the polypeptide can be used to screen for, select, or produce anti-C3d antibodies. Thus, provided herein is a method of screening, selecting, or producing anti-C3d antibodies by contacting one or more antibodies or antibody fragments (e.g., a library, such as a phage display library) with the polypeptide and selecting an antibody or antibody fragment that binds to the polypeptide. The method can comprise repeatedly performing the contacting and selection steps (e.g., panning) using the polypeptide and selecting those antibodies or antibody fragments that exhibit the greatest affinity for the polypeptide. Specific techniques for panning antibody libraries using polypeptides are known in the art. For instance, the polypeptide can be used in conjunction with panning phage display libraries.

The polypeptides also can be used to elicit an immunogenic response in a mammal. Thus, provided herein is a method of eliciting an immunogenic response in a mammal by administering the polypeptide to a mammal. The immunogenic response can be for any purpose, such as for therapy or for the production and subsequent harvesting of antibodies.

Also provided is a nucleic acid encoding the polypeptide, optionally in a vector. The vector can be any of those described herein with respect to the nucleic acid encoding the antibody of the invention.

The following examples further illustrate the invention but, of course, should not be construed as in any way limiting its scope.

### EXAMPLES

### Example 1

This example demonstrates the production and characterization of antibodies of an aspect of the invention.

### Patient samples and cell lines

Peripheral blood mononuclear cells (PBMCs) and bone marrow samples were obtained from CLL patients enrolled in a phase 2 clinical trial of chemoimmunotherapy (NCT 01145209) as previously described (Desai et al., Leuk. Lymphoma, 1-17 (2021)). Samples were prepared by standard Ficoll Hypaque density separation, and cryopreserved until use.

Human B cell lines (SU-DHL-4, SU-DHL-6, RL and JeKo-1) (ATCC) and HBL-2 cells were cultured in RPMI-1640 supplemented with 10% FBS and Pen-Strep-Glutamine (Gibco, Thermo Fisher Scientific). Human B cell lines were authenticated by the supplier. To authenticate the HBL-2 cell line genomic DNA was subjected to CNV finger printing by multiplex PCR and the PCR products were resolved in an agarose gel. Reactions were scored using a binary system: 0 for undetected alleles and 1 for detected alleles. The uniquely identified pattern of bands for HBL-2 cell line matched to that of record in the Staudt's laboratory cell line data base. Multiple vials of each cell line were frozen at an early passage and stored in liquid nitrogen. When needed, the cell lines were thawed, expanded and kept in continuous culture for no more than 1-2 months.

### Generation of rabbit Fab libraries by phage display and rabbit/human chimeric mAbs

Two b9 allotype rabbits were immunized with 100 µg hC3d in Freund's complete adjuvant (FCA), followed by three booster injections with 50 µg hC3d in FCA every three weeks. The serum antibody response to hC3d was monitored during the process by ELISA. Spleen and bone marrow were collected five days after the last boost and separately processed for total RNA preparation and RT-PCR amplification of rabbit V_{κ}, V_{λ}, and V_{H} (rbV_{κ}, rbV_{λ}, and rbV_{H}) encoding cDNA using established protocols and primers. A degenerate reverse primer rbIgG-C_{H}1-R (5'gaagactgaYggagccttaggttg3', Y = t or c, SEQ ID NO: 41) binding to the 5' end of the rabbit IgG constant domain C_{H}1 was used to enrich all rbV_{H} encoding sequences in the secondary immune response antibody repertoire. Rabbit (rb) V_{κ}/human (hu) C_{κ}/rbV_{H} and rbV_{λ}/huC_{λ}/rbV_{H} segments, respectively, were assembled and cloned into phage display vector pC3C as described (Peng et al., J. Mol. Biol., 429: 2954-2973 (2017)). Transformation of *E*. *coli* strain ER2738 (Lucigen Corporation, Middleton, WI) by electroporation yielded approximately 8.6 x 10⁸ and 2.6 x 10⁹ independent transformants for phagemid library κ and λ, respectively. Using VCSM13 helper phage (Agilent Technologies, Savage, MD), the phagemid libraries were converted to phage libraries and stored at -80 °C. Phages displaying the immune rabbit antibody library were selected for binding to zymosan-deposited C3 fragments (C3b/iC3b/C3d) in the presence of 50% NHS to exclude clones that bind full-length, soluble C3. Phage library κ and A were re-amplified using ER2738 and mixed equally before four rounds of panning against native C3 fragments deposited on zymosan. Starting from the third round, panning was performed with or without 50% pooled NHS (PNHS) when phage was incubated with zymosan particles. Following selection, supernatants of IPTG-induced bacterial clones were analyzed by zymosan ELISA or flow cytometry. Selected clones were identified by DNA fingerprinting with *Alu*I, and the V_{L} and V_{H} sequences of unique clones were determined by DNA sequencing.

Phages displaying the immune rabbit antibody library were selected for binding to zymosan-deposited C3 fragments (C3b/iC3b/C3d) in the presence of 50% NHS to exclude clones that bind full-length, soluble C3. Selected clones were identified by DNA fingerprinting with *Alu*I, and the LV and HV sequences of unique clones were determined by DNA sequencing (see Table 1).

### Surface plasmon resonance

Kinetic and thermodynamic parameters characterizing the binding of purified Fabs to hC3d or mC3d was performed by surface plasmon resonance (SPR) in a Biacore X100 instrument (GE Healthcare). Using mouse anti-human IgG C_{H}2 mAb immobilized on a CM5 sensor chip, hFc-hC3d or hFc-mC3d fusion proteins were captured at a density <400 RU. Each sensor chip included an empty flow cell for instantaneous background subtraction. All binding assays used 1x HBS-EP+ running buffer (10 mM HEPES, 150 mM NaCl, 3 mM EDTA (pH 7.4), and 0.05% (v/v) Surfactant P20) at a flow rate of 30 µL/min. All Fabs were injected at five different concentrations (50, 25, 12.5, 6.25, 3.125 nM), and the lowest concentration was tested in duplicates. The sensor chips were regenerated with 3 M MgCl₂ without any loss of binding capacity. Calculation of association (*kₒₙ*) and dissociation (*k_{off}*) rate constants were based on a 1:1 Langmuir binding model. The equilibrium dissociation constant (*K*_{D}) was obtained by the formula K_{D} = *k_{off}*/*kₒₙ*.

### Flow cytometry and histology

Flow cytometry on PBMC, cell lines, and zymosan beads were performed on Canto-II or LSR II instruments (BD Biosciences) and data were analyzed using FlowJo software (Version 10).

Anti-C3d mAbs: C8xi (mouse/human chimeric) and Ab2, Ab5 (rabbit/human chimeric) were HPLC purified. These mAbs and human IgG (isotype control) were conjugated with AlexaFluor-488 or -647 using Protein Labeling Kits (Thermo Fisher Scientific Company, Pittsburg, PA).

Bone marrow biopsies obtained as part of the clinical treatment study were stained with Hematoxylin & Eosin (H&E), CD79a (mAb SP18, Roche Diagnostics), and CD20 (mAb L26, Cell Marque) and reviewed using Olympus microscope.

### Deposition of C3b / iC3b / C3d on zymosan particles and JeKo-1 cells

To deposit C3 fragments (C3b, iC3b and C3d), preactivated zymosan particles were incubated with 50% NHS or with serum from cynomolgus macaques or mice for 30 min at 37 °C (DiLillo et al, Mol. Immunol., 43: 1010-9 (2006)). To deposit mostly C3b, the reaction was stopped after 1 min by adding 10 volumes of GVB-EDTA buffer. Zymosan particles incubated with sera in the presence of 50% GVB-EDTA served as negative control. To deposit C3 fragments on JeKo-1 cells, they were incubated with 10 µg/mL rituximab in the presence of 50% C5△NHS for24 hours.

### Flow cytometry and ELISA with zymosan particles

C3-opsonized zymosan particles (1e6) were incubated with 100 µL of 1 ug/mL His-tagged Fabs in a 96-well V-bottom plate at 37 °C for 1 h. After three washes with PBST (0.05% v/v Tween 20 in PBS), 100 µL of mouse anti-His tag mAb conjugated to horse radish peroxidase (HRP) (R&D Systems, Minneapolis, MN) at 1:1000 dilution in 1% (w/v) BSA / DPBS was added to detect bound Fabs using 2,2'- azino-bis (3-ethylbenzthiazoline)-6-sulfonic acid (Roche/Milipore Sigma, St. Louis, MO) as substrate. The absorbance was measured at 405 nm using a SpectraMax M5 microplate reader and SoftMax Pro software (Molecular Devices, LLC, San Jose, CA). To test for the potential of C3 in NHS to block binding of the mAbs to C3-fragment opsonized zymosan, serial dilutions of NHS were premixed with the opsonized zymosan particles prior to addition of his-tagged Fabs and following the procedure described above.

The binding of rabbit antibodies (Fabs) was detected by flow cytometry following the same procedure described above except using a mouse anti-His tag mAb conjugated to AF488 (Qiagen, Valencia, CA). To determine the influence of soluble C3 fragments, 0.5 µg/mL anti-C3d mAbs conjugated with AF488 or 647 were premixed with soluble C3b, iC3b or C3d at different concentrations for 30 min before adding to the C3-fragment opsonized zymosan particles.

### Cell surface binding of C3 fragments

Frozen PBMC obtained from treatment naive patients with CLL were thawed and washed in FACS buffer (PBS containing 2% heat inactivated FBS) and incubated at 4 °C for 45 min in the absence or presence of 10 µg/mL trastuzumab, rituximab, or ofatumumab. After removing unbound Abs by washing, the cells were incubated in the presence of 50% (v/v) C5△NHS or NMS for 2 h at room temperature for deposition of C3 fragments. Cell surface bound C3 fragments were detected by flow cytometry using AF647-anti-C3d mAbs (C8xi, Ab2, or Ab5). AF647-human IgG served as an isotype control. The same method was used to detect cell surface binding of C3 fragments on B cell lines. Mean fluorescence intensity (MFI) or molecules of equivalent soluble fluorochome (MESF) values were determined using AF647 calibration beads (Bangs Laboratories, Fishers, IN) or Rainbow beads (Spherotech, Inc., Lake Forest, IL) and the lot-specific analysis templates provided by the respective manufacturers (Beurskens et al., J. Immunol., 188: 3532-41 (2012)).

### Antibody dependent cellular phagocytosis (ADCP)

Monocyte-derived macrophages (MDM) were derived from elutriated monocytes from healthy donors (Department of Transfusion Medicine, NIH) and used as effectors as described (Church et al., Clin. Exp. Immunol., 183: 90-101 (2016)).

Cryopreserved CLL PBMC were washed, labeled with violet proliferation dye 450 (VPD-450), incubated with OFA or TRA in the presence of C5△NHS and then opsonized with 10 µg/mL of indicated anti-C3d mAbs at 4 °C for 45 min. Unbound Abs were removed by washing and used as target cells. The effectors and targets were cocultured at 2:1 ratio for 3 h at 37 °C, resuspended in 100 µL FACS buffer and stained with CD11b-PE and CD14-PE Ab cocktail (Miltenyi Biotech Inc. Auburn, CA) for 45 min at 4 °C to identify macrophages. After a final wash, the cells were resuspended in FACS buffer and analyzed by flow cytometry. The effectors (PE+), targets (VPD-450+) and the phagocytosed targets (double positives) were identified. The ADCP was determined as % phagocytosis = (double positive cells / total targets) x 100.

### Whole blood assay for the binding of C3 fragments

To determine potential deposition of C3 fragments (generated by ofatumumab-mediated complement activation) on various blood cell populations, whole blood from healthy donors and patients with CLL was obtained in EDTA tubes. The blood was washed three times in PBS and then resuspended in RPMI-10. One milliliter of cells (5 million) was incubated with 1 mL of C5△NHS (50% v/v) in the presence ofatumumab (20 ug/mL) for 30 min at 37 °C. As controls, whole blood cells were cultured in the presence of ofatumumab alone, C5△NHS alone or C5△NHS, OFA and 25 mM EDTA. The cells were harvested and after removal of the majority of red blood cells (RBCs) using ACK lysing buffer (Quality Biological, Gaithersburg, MD), the remaining cells were washed three times in DPBS (1,800 rpm for 10 min at ambient temperature). This procedure retained all the blood cells including platelets and a sufficient number of RBCs in the sample for subsequent analysis. The cells were incubated with FcR blocking reagent (Miltenyi Biotec Inc., Germany) for 15 min on ice and then stained for 40 min on ice with the following fluorochrome conjugated mAbs (BioLegend Inc., San Diego, CA and BD Biosciences, San Jose, CA) : CD3-BUV496, CD14-BV421, CD15-BV650, CD19-PE-Cy7, CD45-FITC, CD61-PE, CD235a-BUV395, Aqua fluorescent dye 405, and AlexaFluor 647 conjugated anti-C3d mAb (Ab2 or Ab5). The cells were washed with 4 mL of PBS and fixed with 1% paraformaldehyde. Multicolor flow cytometry was performed in a Fortessa LSR (Becton Dickinson, San Jose, CA) and the data was analysed using FlowJo v10.7 software (Tree Star, Inc.) to determine the binding of C3 fragments to different cell populations based on their phenotype markers. Modest levels of C3 fragments bound to blood monocytes and granulocytes are unlikely due to FcyR binding of the anti-C3d mAb because similar results were obtained in experiments using F(ab')2 preparations conjugated to AF647 or blocking of FcyR with excess human IgG.

### Murine xenograft models

Animal experiments were conducted on a protocol approved by the NIH Institutional Animal Care and Use Committee. Mice were purchased from Jackson Laboratories. For lymphoma xenografts, Balb/c-SCID were s.c. injected with cell lines (HBL-2, SU-DHL-6, SU-DHL-4, RL, and MM1-R). Depending on the cell line and number of cells injected, >50% of mice showed palpable or measurable tumors between 7 and 14 days.

To determine whether the anti-C3d mAbs used in the treatment of xenografted mice caused organ damage, H&E stained slides of kidney, lung, heart, spleen, and eye of 2-3 mice from each treatment group were analyzed on an Olympus BX41 microscope by an expert veterinarian pathologist. No gross changes in morphology were observed in any of the organs analyzed.

### Results

### Derivation of rabbit anti-C3d mAbs

To more broadly test the strategy in murine models, anti-C3d mAbs that cross-reactive to mouse and human C3d were created. Using hC3d as the immunogen, rabbit mAbs were created by phage display technology. Several unique mAbs based on the amino acid sequences of their complementarity-determining regions were identified (Table 1 with Kabat CDRs identified in Table 2).

**Table 1**

| Antibody Chain | Sequence | SEQ ID NO |
|---|---|---|
| Ab1VH | QSVEESRGRLVTPGTPLTLTCTVSGIDLSSSAITWVRQAPGKGLEWI GMISTDGATYYANWAKGRFTFSKTSTTADLKITSPTTEDTATYFCAR GLTNFSIWGPGTLVTVSS | 1 |
| Ab1 VL | QPVLTQSPSASAALGASAKLTCTLSSAHKTYTIAWYQQQPGESPRYL MLLKSDGSYTRGPGVPDRFSGSSSGADRYLIIPSVQADDEAGYYCG ADYNGGYVFGGGTQLTVTG | 2 |
| Ab2 VH | QSVEESGGRLVTPGTPLTLTCTVSGFSLSSYAMTWVRQAPGKGLEW IGVIGTSGMRYYANWAKGRFTISKTSTTVDLKITSPTTEDTATYFCAR ANFYVGNDESTDAFDPWGPGTLVTVSS | 3 |
| Ab2 VL | ALVMTQTPPSLSASVGETVRIRCLASEDIYNTISWYQQKPGKPPTLLI YGASNLKSGVPPRFSGSGSGTDYTLTIGGVQSEDAATYYCLGGYSY SSTGLTFGAGTKVEIK | 4 |
| Ab3 VH | QKQLVESGGRLVTPGGSLTLTCTISGIDLNTYGMTWVRQAPGKGLEY IGIIVSSGNTYYANWAQGRFTISKTSTTVDLKMTSLTASDTATYFCAR GLGRAYSSGYGMDGWGPGTLVTVSS | 5 |
| Ab3 VL | DPVLTQTPSSTSAAVGGTVTIKCQASQNIYTSLAWYQQKPGQPPKLLI YGASNLASGVPSRFKGSRSGTEYTLTISGVEREDAATYYCLGSYTQI DTTFGGGTKLEIK | 6 |
| Ab4 VH | QSLEESGGRLVTPGTPLTLTCTVSGFSLSRYTMAWVRQAPGKGLEYI GWIDSSGSTYYASWAKGRFTISKTSSTTVDLKMTSLTTEDTATYFCA SGNDGGDPLYYGMDPWGPGTLVTVSS | 7 |
| Ab4 VL | DGVMTQTPSSTSAAVGGTVTIKCQASQSIRSWLSWYQQKPGQPPKL LIYEASKLASGVPSRFKGSRSGTEYTLAISGVQREDAATYYCLGSIAS DDTAFGAGTNVEIK | 8 |
| Ab5 VH | QSLEESGGRLVTPGTPLTLTCTVSGFSLSTYTLGWVRQAPGKGLEYI GWIDSSGSAYYASWAKGRFTISKASSTTVDLKMAGLTTEDTATYFCA SGNTGGDPLYYGMDPWGPGTLVTVSS | 9 |
| Ab5 VL | DPVLTQTPSSTSAAVGGTVTIKCQASQNIGRWLAWYQQKPGQPPKL LIYRASDLASGVPSRFKGSGSGTQFTLTISGVQREDAATYYCLGSYS SSDSAFGGGTEVVVK | 10 |

**Table 2**

| **HCDR1** | **HCDR2** | **HCDR3** | **LCDR1** | **LCDR2** | **LCDR3** |
|---|---|---|---|---|---|
| **Ab1 (λ)** | | | | | |
| SSAIT (SEQ ID NO: 11) | MISTDGATY YANWAKG (SEQ ID NO: 12) | GLTNFSI (SEQ ID NO: 13) | TLSSAHKTY TIA (SEQ ID NO: 14) | LKSDGSYT R (SEQ ID NO: 15) | GADYNGGY V (SEQ ID NO: 16) |

| **Ab2 (κ)** | | | | | |
|---|---|---|---|---|---|
| SYAMT (SEQ ID NO: 17) | VIGTSGMRY YANWAKG (SEQ ID NO: 18) | ANFYVGND ESTDAFDP (SEQ ID NO: 19) | LASEDIYNTI S (SEQ ID NO: 20) | GASNLKS (SEQ ID NO: 21) | LGGYSYSS TGLT (SEQ ID NO: 22) |

| **Ab3 (κ)** | | | | | |
|---|---|---|---|---|---|
| TYGMT (SEQ ID NO: 23) | IIVSSGNTYY ANWAQG (SEQ ID NO: 24) | GLGRAYSS GYGMDG (SEQ ID NO: 25) | QASQNIYTS LA (SEQ ID NO: 26) | GASNLAS (SEQ ID NO: 27) | LGSYTQIDT T (SEQ ID NO: 28) |

| **Ab4 (κ)** | | | | | |
|---|---|---|---|---|---|
| RYTMA (SEQ ID NO: 29) | WIDSSGSTY YASWAKG (SEQ ID NO: 30) | GNDGGDPL YYGMDP (SEQ ID NO: 31) | QASQSIRS WLS (SEQ ID NO: 32) | EASKLAS (SEQ ID NO: 33) | LGSIASDDT A (SEQ ID NO: 34) |

| **Ab5 (κ)** | | | | | |
|---|---|---|---|---|---|
| TYTLG (SEQ ID NO: 35) | WIDSSGSA YYASWAKG (SEQ ID NO: 36) | GNTGGDPL YYGMDP (SEQ ID NO: 37) | QASQNIGR WLA (SEQ ID NO: 38) | RASDLAS (SEQ ID NO: 39) | LGSYSSSD SA (SEQ ID NO: 40) |

Binding of the antibodies to human, cynomolgus, and mouse C3d was characterized. Four mAbs showed the desired binding to C3d from all 3 species, with Ab5 and Ab2 binding strongly to both mouse and human C3d (Figures 1 and 4). Ab4 and Ab3 sharing sequence similarities with Ab5 (Table 1) bound both human and mouse C3d weaker than Ab5 (Figure 1, Table 3). On SPR, C8xi and the rabbit mAbs Ab2 and Ab5 showed independent binding to immobilized hC3d. Ab4 showed no additional binding over Ab5, suggesting that the two mAbs bind overlapping epitopes, consistent with their sequence similarities (Figure 2).

**Table 3**

| Clones | C8xi | Ab2 | Ab5 | Ab4 | Ab3 | Ab2 | Ab5 | Ab4 | Ab3 |
|---|---|---|---|---|---|---|---|---|---|
| Antigen | hC3d | hC3d | hC3d | hC3d | hC3d | mC3d | mC3d | mC3d | mC3d |
| [Fab]^{hi} (nM) | 100 | 20 | 50 | 50 | 50 | 50 | 50 | 200 | 200 |
| kon (10⁴ M⁻¹ s⁻¹) | 85.1 | 16.2 | 14.1 | 9.09 | 9.46 | 19.3 | 24.8 | 6.06 | 23.2 |
| koff (10⁻³ s⁻¹) | 57.0 | 0.54 | 0.14 | 0.28 | 1.26 | 1.74 | 5.68 | 8.01 | 22.7 |
| Kd (nM) | 67.0 | 3.35 | 0.99 | 3.10 | 13.3 | 9.04 | 22.9 | 132 | 97.6 |
| Chi² | 3.5 | 0.43 | 0.51 | 0.27 | 0.64 | 0.39 | 0.52 | 9.52 | 2.00 |

Next, the degree of cross-reactivity with full length human C3 present in plasma was assessed (Figure 5). C3d binding by Ab5 and Ab2 was only slightly reduced while binding by Ab1 and C8xi was not competed in the presence of human plasma.

To characterize the affinities of the lead mAbs to different C3 fragments, the degree to which purified soluble C3b, iC3b, or C3d could compete with mAb binding to C3 fragments (C3b/iC3b/C3d) deposited on zymosan was tested. The relative specificities were for C8xi: iC3b>C3b>C3d; for Ab2: iC3b=C3b>C3d; and for Ab5: C3d> iC3b>C3b (Figure 3). Thus, the anti-C3d mAbs engage C3 fragments from the first to the last step of the complement cascade. Consistently, the antibodies showed strong binding to zymosan opsonized with C3b (Figure 6) as well as to Jeko cells opsonized in NHS for 2 hours and 24 hours; at the later timepoint processing to C3d is known to be complete (Figure 7). Both C8xi and Ab2 strongly bound CLL cells opsonized in NHS but, as expected, Ab2 but not C8xi bound cells opsonized in NMS (Figure 8). Ab2 and Ab5 avidly bound lymphoma cell lines reacted with ofatumumab in NHS. Interestingly, signal intensities for the anti-C3d mAbs bested staining by anti-CD20 mAbs (Figure 9).

### Effector functions of anti-C3d mAbs

Several lines of evidence indicate that ADCP is the major cellular mechanism of action of therapeutic anti-CD20 mAbs (Zent et al., Antibodies (Basel), 9(4): 68 (2020); VanDerMeid et al., Cancer Immunol. Res., 6: 1150-1160 (2018); Chu et al., J. Cell Sci., 133, 2020)). To compare ADCP between anti-CD20 and anti-C3d mAbs human monocyte-derived macrophages (MDM) was used as effector cells and VPD-450-stained PBMCs from patients with CLL obtained before and on Day 2 of ofatumumab treatment as target cells. After in vitro incubation with mAbs, the percentage of VPD stained cells associated with macrophages by flow cytometry was assessed (Figure 10). As expected, ofatumumab mediated phagocytosis of PBMCs obtained from the patients *before* (Day 1) but not for samples obtained *after* (Day 2) ofatumumab administration. In contrast, anti-C3d mAbs mediated phagocytosis of PBMCs obtained on Day 2 but not on Day 1 (Figure 11).

Deposition of C3 fragments on cells other than CD20+ B cells, could lead to off-target effects. To test whether anti-C3d mAbs preserve the specificity of the anti-CD20 mAb, ofatumumab was added to whole blood assays. While B cells became abundantly opsonized with C3 fragments, other cell types showed no or only minimal anti-C3d staining over background (Figures 12 and 13).

The combination of rabbit/human chimeric anti-C3d with anti-CD20 mAbs improves survival in lymphoma xenograft models

The rabbit/human chimeric mAbs were used to further explore combinations of anti-CD20 with anti-C3d mAbs in murine lymphoma models. In the MCL xenograft model, mice were randomized to no mAb administration, ofatumumab alone, ofatumumab with Ab2, ofatumumab with Ab5, and trastuzumab with Ab5. Median survival of untreated mice was 21.5 days, compared to 35 days for mice treated with ofatumumab alone (*P*=.001) and 63 days for mice receiving the combination of ofatumumab with an anti-C3d mAb (*P*=.012 for comparison to ofatumumab alone, and *P*=.0001 for comparison with untreated mice) (Figure 14). Half of the lymphoma carrying mice treated with the combination survived for over 300 days. In contrast, all other mice reached study endpoint before or by day 51. Both anti-C3d mAbs, Ab5 and Ab2, provided comparable survival benefits (Figures 15 and 16).

Next, anti-C3d mAbs were tested in models of diffuse large B-cell lymphoma (DLBCL) and follicular lymphoma (FL) using SU-DHL-6 and RL cell lines, respectively. In the SU-DHL-6 model, 40 mice were randomized to no treatment, or 8 weekly injections of anti-CD20 mAb, either rituximab or ofatumumab, combined with trastuzumab or an anti-CD20 mAb combined with an anti-C3d mAb, either Ab2 or Ab5 (Figure 17). Treatment was initiated on day 7 when most mice had formed palpable tumors. Median survival was 42 days for untreated mice, 114 days for mice treated with an anti-CD20 mAb (*P*<.0001), and not reached for the combination of an anti-CD20 with an anti-C3d mAb (Figure 17; P<.0001 for comparison to untreated; *P*=.008 for comparison to anti-CD20 with trastuzumab). Tumor-free survival at 6 months was 20% for mice treated with an anti-CD20 mAb combined with trastuzumab and 75% for mice treated with the combination of mAbs against CD20 and C3d. There was no difference in outcome depending on which anti-CD20, either rituximab or ofatumumab (Figures 18 and 19), or which anti-C3d mAb, either Ab2 or Ab5 (Figures 20 and 21), was used.

The RL cell line, derived from rituximab resistant FL, has been used to model anti-CD20 mAb resistance (Dalle et al., Clin. Cancer Res., 15: 851-7 (2009)). Mice were injected s.c. with 10 million RL cells. After 21 days, weekly injections of 20 mg/kg rituximab alone or a combination of 10 mg/kg rituximab and 10 mg/kg anti-C3d mAb were started. Median survival from the start of rituximab therapy was 18 days versus 11 days for untreated mice (*P*=.21), and all mice in these 2 cohorts were euthanized by day 18 (Figure 22). The combination of rituximab with an anti-C3d mAb (Ab2 or Ab5) improved median survival to 29 days (*P*=.004), with one mouse surviving for more than 2 months.

In the SU-DHL-4 DLBCL model median survival of untreated mice was 92 days and all mice were euthanized before day 100. At 6 months, survival was 60% for mice treated with rituximab single-agent (*P*=.013) and 80% for the combination of rituximab with an anti-C3d mAb (*P*<.001). There was no statistically significant difference in survival between the treatment groups (*P*=.39; Figure 23). The long-term survivors were used to investigate whether addition of the anti-C3d mAb was associated with adverse events. There was a trend for higher median weight between strain controls and survivors in the rituximab compared to the combination arm (31.9gm vs 29.3gm, *P*=.052). Serum creatinine, reflecting kidney function, was normal and showed little variability between mice (Table 4). At the termination of the experiment, immunohistochemical stains prepared from different organs including kidneys and eyes, where complement deposition has been implicated in human disease, revealed no pathology (Figure 24).

**Table 4**

| MOUSE | TREATMENT | WEIGHT (GM) | CREATININE (0.2 - 1.3) |
|---|---|---|---|
| Y614 | None* | 32.0 | 0.26 |
| Y615 | None* | 28.5 | 0.26 |
| Y616 | None* | 30.3 | 0.32 |
| 2022 | RTX | 31.8 | 0.3 |
| 2023 | RTX | 33.4 | 0.3 |
| 2024 | RTX | 33.5 | 0.3 |
| 2027 | RTX + Ab2 | 30.2 | 0.4 |
| 2028 | RTX + Ab2 | 30.8 | 0.3 |
| 2030 | RTX + Ab2 | 28.9 | 0.3 |
| 2031 | RTX + Ab2 | 28.1 | 0.2 |
| 2032 | RTX + Ab5 | 32.2 | 0.3 |
| 2034 | RTX + Ab5 | 27.1 | 0.4 |
| 2035 | RTX + Ab5 | 23.4 | 0.5 |
| 2036 | RTX + Ab5 | 29.6 | 0.3 |

Fc-receptor expressing phagocytes are key to the elimination of antibody opsonized tumor cells but also give rise to antigen-loss through trogocytosis (Taylor et al., Blood, 125: 762-6 (2015); Lindorfer MA, Taylor RP, Antibodies (Basel), 11 (2022); Behrens et al., Immunol. Rev., 314(1): 280-301 (2022)). Most therapeutic mAbs used in hematologic malignancies also fix complement and mediate rapid deposition of C3 fragments that remain covalently attached to target cells. In patients treated with anti-CD20 mAbs, these B cell-bound C3 fragments are quickly (less than 30 min) degraded to C3d (Kennedy et al., J. Immunol., 172: 3280-8 (2004)). C3d opsonized cells persist for weeks after antibody infusion (Beurskens et al., J. Immunol., 188: 3532-41 (2012)) indicating that innate effector functions alone are insufficient to clear these cells. Here, it was tested whether a C3d targeting mAb given after or in conjunction with a complement fixing primary mAb, could deliver a "one-two" punch and increase efficacy of mAb therapy.

In the SU-DHL-4 DLBCL model both rituximab alone and rituximab combined with an anti-C3d mAb Ab2 or Ab5 "cured" the majority of mice with no difference in efficacy; providing an opportunity to study the relative tolerability of the two regimens. Overall survival was the same, with mice in the combination arm showing a trend for lower body weight. However, serum creatinine and histologic examination of key organs did not demonstrate any pathology. In conclusion, no detrimental effects of anti-C3d administration in these animals was uncovered. Likewise, whole blood assays demonstrated strong binding of anti-C3d mAbs primarily to B cells, with weak binding to monocytes and granulocytes but not to other cell populations. Thus, antiC3d mAbs preserved the target selectivity of the primary anti-CD20 mAb, easing concerns about off-target effects. It is also worth considering that limited reactivity of therapeutic mAbs with non-tumor cells may be permissible. For example, non-withstanding expression of CD38 on red cells, NK cells, and some T cells, daratumumab has been successfully integrated into treatment for MM (Sullivan et al., Blood, 129: 3033-3037 (2017); Krejcik et al., Blood, 128: 384-94 (2016)).

This example focused on anti-CD20 mAbs. Rituximab is commonly used in NHL indications and is now also widely available as biosimilar. In the CLL clinical trial, ofatumumab was use (Hillmen et al., Lancet, 385: 1873-83 (2015)). Ofatumumab, a fully human anti-CD20, is now used primarily for the treatment of multiple sclerosis (Costa et al., Expert Rev. Clin. Immunol., 18: 105-114 (2022)). Importantly, rituximab and ofatumumab had comparable activity in the xenograft models. Given that most anti-cancer antibodies fix complement, the approach described herein can be applied across clinical indications. For example, daratumumab, the first mAb approved for the treatment of multiple myeloma, shares important similarities with anti-CD20 mAbs, including strong complement deposition and antigen-loss on tumor cells due to trogocytosis that contributes to treatment failure (Nijhof et al., Blood, 128: 959-70 (2016); Krejcik et al., Clin. Cancer Res., 23: 7498-7511 (2017)).

Although C3d was used as the immunogen, all of the anti-C3d mAbs described herein also bind to C3b and to iC3b. C3b and iC3b are intermediary fragments upstream of C3d. Therefore, it is possible that when anti-C3d and anti-CD20 mAbs are given together, binding to early C3 activation fragments (C3b, iC3b) deposited on the target cells will also occur. In the xenograft models, concurrent administration of mAbs was effective, suggesting that binding of early fragments would not interfere with anti-tumor targeting. Importantly, all three lead mAbs are not or minimally competed by full length C3. Given that C3d remains covalently bound to complement opsonized cells, the timing of anti-C3d mAb administration would not appear to be critical.

This example provides proof-of-principle for the utility of C3d-targeting to enhance efficacy of mAb therapy. Administration of anti-C3d mAbs concurrently or sequentially with a primary complement-fixing mAb can be tested. Here human IgG1 chimeric mAbs werre used that could be Fc engineered to enhance complement activation and C3d deposition (Diebolder et al, Science, 343: 1260-3 (2014)), or increase FcγR affinity for more efficient phagocytosis (Kellner et al, Methods, 65: 105-13 (2014)). Likewise, anti-C3d targeting could be engineered into T-cell engaging bispecific antibodies. Thus, anti-C3d mAbs could rapidly translate desired effector functions to existing therapeutic antibodies across a spectrum of therapeutic indications.

### Example 2

This example demonstrates effective combination treatments using anti-C3d mAbs of an aspect of the invention with an anti-CD38 mAb.

The mAb daratumumab (DARA) targeting CD38 can induce minimal residual disease negative remissions in patients with multiple myeloma (MM). However, the majority of patients subsequently relapse or become refractory to treatment (RRMM). Rapid reduction of surface CD38 levels in MM cells following DARA infusion due to trogocytosis by monocytes and granulocytes leads to reduced complement dependent cytotoxicity (CDC) and antibody dependent cellular cytotoxicity (ADCC), effector functions that rely on antigen density. High expression of complement regulatory proteins, CD55 and CD59 on MM cell surface can also impede CDC activity. Therefore, modalities to overcome these limitations and improve clinical outcome for patients with RRMM are needed.

Daratumumab is a strong activator of complement. While a subset of cells may be lysed by the membrane attack complex, all cells reacted with daratumumab are opsonized by C3d, the terminal component of C3. C3d remains covalently attached at the cell surface. Thus, the newly acquired membrane bound C3d constitutes a target for additional intervention, particularly when the cell surface CD38 is lost or low following treatment with DARA. Two high affinity anti-C3d mAbs (clones Ab2 and Ab5 with kD values 3.35 nM and 0.99 nM, respectively) that showed reactivity against both human and mouse C3d were selected for further study. To deposit C3d on myeloma cell line (MM1-R) without activating CDC, the cells were reacted with DARA in C5 depleted normal human serum. Both Ab2 and Ab5 bound to the C3d opsonized cells (Figures 25A and 25B). Based on SPR analysis these two mAbs do not compete for C3d binding suggesting that they recognize distinct epitopes. In normal human serum, the anti-C3d mAbs enhanced killing by CDC of DARA-opsonized target cells compared to DARA alone (P<0.0001). The non-targeting IgG1 isotype control mAb trastuzumab (TRA) did not bind C3d or enhance DARA-mediated killing.

To test the combination of DARA with anti-C3d mAbs in vivo, Balb/c-SCID mice bearing subcutaneous (s.c.) MM1-R tumors were used. First, using clones Ab2 and Ab5, it was demonstrated *in vivo* binding of mouse C3d on MM1-R tumor cells harvested 24 h after a single intraperitoneal (i. p.) injection of DARA (Figure 26). Next, mice were challenged s. c. with MM1-R cells and randomly divided into the following treatment groups: 20 mg/kg DARA single agent (n=7), or combination treatments of 10 mg/kg of each of two mAbs: DARA + TRA (n=10), DARA + Ab2 (n=13), DARA + Ab5 (n=13) (Figure 27). The mAbs were given via i. p. injections weekly starting on day 7 post tumor challenge and continued six weekly followed by two biweekly injections (up to 8 injections total). Palpable tumors were noticed in mice between days 7 and 14, and tumor growth was monitored by caliper measurements twice weekly. Mice were euthanized when tumors reached pre-defined limits. All the mice that received DARA + TRA showed progressive tumor growth with median survival of 43 days and all mice had to be euthanized by day 50. Doubling the dose of DARA to 20mg/kg slightly increased median survival to 43 days (P = 0.12 vs DARA+TRA). Two of 7 mice that received DARA alone (at 20 mg/kg) survived to day 175. Median survival for mice treated with DARA (10mg/kg) plus an anti-C3d mAb was 162 days (P = 0.006 for comparison to DARA 20mg/kg), and 13 of 26 (50%) mice in this cohort survived tumor-free beyond 200 days. There was no statistically significant difference between groups treated with Ab2 or Ab5 in combination with DARA (P = 0.23). All the surviving mice showed no evidence of tumor, weight loss or other morbidity indicating that they are essentially tumor-free.

In summary, these results demonstrate that combination treatment of DARA with antiC3d mAbs improves efficacy over single agent DARA leading to long-term survival in the MM.1R xenograft model. The combination of complement targeting mAbs with DARA could improve clinical efficacy and overcome resistance to single agent therapy.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

The following are embodiments of the invention:
1. An anti-C3d antibody or antibody fragment comprising:
   (a) a heavy chain immunoglobulin variable region comprising SEQ ID NO: 1, or at least the CDRs thereof; and a light chain immunoglobulin variable region comprising SEQ ID NO: 2, or at least the CDRs thereof;
   (b) a heavy chain immunoglobulin variable region comprising SEQ ID NO: 3, or at least the CDRs thereof; and a light chain immunoglobulin variable region comprising SEQ ID NO: 4, or at least the CDRs thereof;
   (c) a heavy chain immunoglobulin variable region comprising SEQ ID NO: 5, or at least the CDRs thereof; and a light chain immunoglobulin variable region comprising SEQ ID NO: 6, or at least the CDRs thereof;
   (d) a heavy chain immunoglobulin variable region comprising SEQ ID NO: 7, or at least the CDRs thereof; and a light chain immunoglobulin variable region comprising SEQ ID NO: 8, or at least the CDRs thereof; or
   (e) a heavy chain immunoglobulin variable region comprising SEQ ID NO: 9, or at least the CDRs thereof; and a light chain immunoglobulin variable region comprising SEQ ID NO: 10, or at least the CDRs thereof.
2. The anti-C3d antibody or antibody fragment according to embodiment 1 comprising:
   (a) a heavy chain immunoglobulin variable region comprising SEQ ID NO: 1; and a light chain immunoglobulin variable region comprising SEQ ID NO: 2;
   (b) a heavy chain immunoglobulin variable region comprising SEQ ID NO: 3; and a light chain immunoglobulin variable region comprising SEQ ID NO: 4;
   (c) a heavy chain immunoglobulin variable region comprising SEQ ID NO: 5; and a light chain immunoglobulin variable region comprising SEQ ID NO: 6;
   (d) a heavy chain immunoglobulin variable region comprising SEQ ID NO: 7; and a light chain immunoglobulin variable region comprising SEQ ID NO: 8; or
   (e) a heavy chain immunoglobulin variable region comprising SEQ ID NO: 9; and a light chain immunoglobulin variable region comprising SEQ ID NO: 10.
3. The anti-C3d antibody or antibody fragment of embodiment 1 comprising:
   (a) a heavy chain immunoglobulin variable region comprising
      CDRH1 consisting of SEQ ID NO: 11,
      CDRH2 consisting of SEQ ID NO: 12,
      CDRH3 consisting of SEQ ID NO: 13,
      and a light chain immunoglobulin variable region comprising
      CDRL1 consisting of SEQ ID NO: 14,
      CDRL2 consisting of SEQ ID NO: 15,
      CDRL3 consisting of SEQ ID NO: 16;
   (b) a heavy chain immunoglobulin variable region comprising
      CDRH1 consisting of SEQ ID NO: 17,
      CDRH2 consisting of SEQ ID NO: 18,
      CDRH3 consisting of SEQ ID NO: 19,
      and a light chain immunoglobulin variable region comprising
      CDRL1 consisting of SEQ ID NO: 20,
      CDRL2 consisting of SEQ ID NO: 21,
      CDRL3 consisting of SEQ ID NO: 22,
   (c) a heavy chain immunoglobulin variable region comprising
      CDRH1 consisting of SEQ ID NO: 23,
      CDRH2 consisting of SEQ ID NO: 24,
      CDRH3 consisting of SEQ ID NO: 25,
      and a light chain immunoglobulin variable region comprising
      CDRL1 consisting of SEQ ID NO: 26,
      CDRL2 consisting of SEQ ID NO: 27,
      CDRL3 consisting of SEQ ID NO: 28;
   (d) a heavy chain immunoglobulin variable region comprising
      CDRH1 consisting of SEQ ID NO: 29,
      CDRH2 consisting of SEQ ID NO: 30,
      CDRH3 consisting of SEQ ID NO: 31,
      and a light chain immunoglobulin variable region comprising
      CDRL1 consisting of SEQ ID NO: 32,
      CDRL2 consisting of SEQ ID NO: 33,
      CDRL3 consisting of SEQ ID NO: 34,
      or
   (e) a heavy chain immunoglobulin variable region comprising
      CDRH1 consisting of SEQ ID NO: 35,
      CDRH2 consisting of SEQ ID NO: 36,
      CDRH3 consisting of SEQ ID NO: 37,
      and a light chain immunoglobulin variable region comprising
      CDRL1 consisting of SEQ ID NO: 38,
      CDRL2 consisting of SEQ ID NO: 39,
      CDRL3 consisting of SEQ ID NO: 40;
   wherein the CDRs are as determined according to Kabat.
4. The antibody or antibody fragment of any of embodiments 1-3, wherein the antibody or antibody fragment is an lgA1, IgA2, IgD, IgE, IgG1, IgG2, IgG3, IgG4, IgM, scFv, IgGΔCH₂, F(ab')2, scFv2CH3, F(ab), scFv4, scFv3, scFv2, dsFv, Fv, scFv-Fc, (scFv)2, diabody, T-body, multispecific antibody, or multivalent antibody.
5. The antibody or antibody fragment of any of embodiments 1-4, wherein the antibody or antibody fragment is conjugated to another molecule.
6. The antibody of embodiment 5, wherein the antibody or antibody fragment is conjugated to a transmembrane region and an intracellular T-cell receptor (TCR) signaling domain to provide a T-body.
7. The antibody of embodiment 5, wherein the anti-C3d antibody or antibody fragment is conjugated to a label.
8. The antibody of embodiment 5, wherein the anti-C3d antibody or antibody fragment is conjugated to a cytotoxic agent or a therapeutic radioisotope.
9. A method of killing a cancer cell having C3d complement protein on the surface thereof, or otherwise treating cancer characterized by surface C3d complement protein, in a subject, the method comprising administering to the subject an anti-C3d antibody or antibody fragment of any of embodiments 1-8.
10. The method of embodiment 9, further comprising inducing the formation of C3d complement protein on the surface of the cancer cell by contacting the cell with an antibody or antibody fragment to a cell-surface protein other than C3d.
11. The method of embodiment 10, wherein the anti-C3d antibody is a multi-specific antibody that is immunospecific for C3d and a cell-surface protein other than C3d, and contacting the cell with an antibody or antibody fragment to a cell-surface protein other than C3d is accomplished by administering the multi-specific antibody.
12. The method of embodiment 10, wherein the method comprises administering to the subject an antibody or antibody fragment that specifically binds to a cell-surface protein other than C3d simultaneously or sequentially in any order with the administration of the anti-C3d antibody or antibody fragment.
13. The method of embodiment 10, wherein the antibody or antibody fragment to a cell surface protein other than C3d is an anti-CD20 antibody or antibody fragment.
14. The method of embodiment 13, wherein the anti-CD20 antibody or antibody fragment is rituximab, ofatumumab, ocrelizumab, veltuzumab, obinutuzumab, PRO131921, or ocaratuzumab.
15. The method of embodiment 13 or 14, wherein the cancer cell is a B-cell expressing CD20.
16. The method of any one of embodiments 9-12, wherein the antibody or antibody fragment to a cell surface protein other than C3d is an anti-CD33 antibody or antibody fragment.
17. The method of embodiment 16, wherein the cancer cell is a myeloid cell.
18. The method of any one of embodiments 9-12, wherein the antibody or antibody fragment that specifically binds to a cell surface protein other than C3d is an anti-CD38 antibody or antibody fragment.
19. The method of embodiment 18, wherein the cancer cell is a plasma cell.
20. The method of any one of embodiments 9-12, wherein the method comprises contacting the cancer cell with an anti-EGFR or anti-ERBB2 antibody or antibody fragment.
21. The method of embodiment 20, wherein the cancer cell is from gut, colon, lung, breast, head, neck, pancreas, ovary, endometrium, or brain tissue.
22. A pharmaceutical composition comprising the antibody or antibody fragment of any of embodiments 1-8.
23. A nucleic acid encoding the antibody or antibody fragment of any of embodiments 1-8, optionally in a vector.
24. A method of preparing an antibody or antibody fragment of any of embodiments 1-8, the method comprising expressing a nucleic acid encoding the antibody or antibody fragment in a cell.
25. A cell comprising the nucleic acid of embodiment 23.
26. The anti-C3d antibody or antibody fragment of any of embodiments 1-8 for treating cancer in a subject.
27. The anti-C3d antibody or antibody fragment according to embodiment 26, wherein the cancer characterized by surface C3d complement protein.
28. The anti-C3d antibody or antibody fragment according to embodiment 26 or 27, wherein the anti-C3d antibody or antibody fragment is for use with an antibody or antibody fragment to a cell-surface protein other than C3d.
29. The anti-C3d antibody or antibody fragment of embodiment 28, wherein the antiC3d antibody is a multi-specific antibody that is immunospecific for C3d and a cell-surface protein other than C3d, and contacting the cell with an antibody or antibody fragment to a cell-surface protein other than C3d is accomplished by administering the multi-specific antibody.
30. The anti-C3d antibody or antibody fragment of embodiment 28, wherein the antibody or antibody fragment to a cell surface protein other than C3d is an anti-CD20 antibody or antibody fragment.
31. The anti-C3d antibody or antibody fragment of embodiments 30, wherein the anti-CD20 antibody or antibody fragment is rituximab, ofatumumab, ocrelizumab, veltuzumab, obinutuzumab, PRO131921, or ocaratuzumab.
32. The anti-C3d antibody or antibody fragment of embodiment 30 or 31, wherein the cancer cell is a B-cell expressing CD20.
33. The anti-C3d antibody or antibody fragment of embodiments 28, wherein the antibody or antibody fragment to a cell surface protein other than C3d is an anti-CD33 antibody or antibody fragment.
34. The anti-C3d antibody or antibody fragment of embodiment 33, wherein the cancer cell is a myeloid cell.
35. The anti-C3d antibody or antibody fragment of embodiment 28, wherein the antibody or antibody fragment that specifically binds to a cell surface protein other than C3d is an anti-CD38 antibody or antibody fragment.
36. The anti-C3d antibody or antibody fragment of embodiment 35, wherein the cancer cell is a plasma cell.
37. The anti-C3d antibody or antibody fragment of embodiment 35, wherein the cancer cell is an LKB1-mutant non-small cell lung cancer cell.
38. The anti-C3d antibody or antibody fragment of embodiment 28, wherein the method comprises contacting the cancer cell with an anti-EGFR or anti-ERBB2 antibody or antibody fragment.
39. The anti-C3d antibody or antibody fragment of embodiment 38, wherein the cancer cell is from gut, colon, lung, breast, head, neck, pancreas, ovary, endometrium, or brain tissue.

## Claims

1. An anti-C3d antibody or antibody fragment comprising:
(a) a heavy chain immunoglobulin variable region comprising SEQ ID NO: 1, or at least the CDRs thereof; and a light chain immunoglobulin variable region comprising SEQ ID NO: 2, or at least the CDRs thereof;
(b) a heavy chain immunoglobulin variable region comprising SEQ ID NO: 3, or at least the CDRs thereof; and a light chain immunoglobulin variable region comprising SEQ ID NO: 4, or at least the CDRs thereof;
(c) a heavy chain immunoglobulin variable region comprising SEQ ID NO: 5, or at least the CDRs thereof; and a light chain immunoglobulin variable region comprising SEQ ID NO: 6, or at least the CDRs thereof;
(d) a heavy chain immunoglobulin variable region comprising SEQ ID NO: 7, or at least the CDRs thereof; and a light chain immunoglobulin variable region comprising SEQ ID NO: 8, or at least the CDRs thereof; or
(e) a heavy chain immunoglobulin variable region comprising SEQ ID NO: 9, or at least the CDRs thereof; and a light chain immunoglobulin variable region comprising SEQ ID NO: 10, or at least the CDRs thereof.

2. The anti-C3d antibody or antibody fragment according to claim 1 comprising:
(a) a heavy chain immunoglobulin variable region comprising SEQ ID NO: 1; and a light chain immunoglobulin variable region comprising SEQ ID NO: 2;
(b) a heavy chain immunoglobulin variable region comprising SEQ ID NO: 3; and a light chain immunoglobulin variable region comprising SEQ ID NO: 4;
(c) a heavy chain immunoglobulin variable region comprising SEQ ID NO: 5; and a light chain immunoglobulin variable region comprising SEQ ID NO: 6;
(d) a heavy chain immunoglobulin variable region comprising SEQ ID NO: 7; and a light chain immunoglobulin variable region comprising SEQ ID NO: 8; or
(e) a heavy chain immunoglobulin variable region comprising SEQ ID NO: 9; and a light chain immunoglobulin variable region comprising SEQ ID NO: 10.

3. The anti-C3d antibody or antibody fragment of claim 1 comprising:
(a) a heavy chain immunoglobulin variable region comprising
CDRH1 consisting of SEQ ID NO: 11,
CDRH2 consisting of SEQ ID NO: 12,
CDRH3 consisting of SEQ ID NO: 13,
and a light chain immunoglobulin variable region comprising
CDRL1 consisting of SEQ ID NO: 14,
CDRL2 consisting of SEQ ID NO: 15,
CDRL3 consisting of SEQ ID NO: 16;
(b) a heavy chain immunoglobulin variable region comprising
CDRH1 consisting of SEQ ID NO: 17,
CDRH2 consisting of SEQ ID NO: 18,
CDRH3 consisting of SEQ ID NO: 19,
and a light chain immunoglobulin variable region comprising
CDRL1 consisting of SEQ ID NO: 20,
CDRL2 consisting of SEQ ID NO: 21,
CDRL3 consisting of SEQ ID NO: 22,
(c) a heavy chain immunoglobulin variable region comprising
CDRH1 consisting of SEQ ID NO: 23,
CDRH2 consisting of SEQ ID NO: 24,
CDRH3 consisting of SEQ ID NO: 25,
and a light chain immunoglobulin variable region comprising
CDRL1 consisting of SEQ ID NO: 26,
CDRL2 consisting of SEQ ID NO: 27,
CDRL3 consisting of SEQ ID NO: 28;
(d) a heavy chain immunoglobulin variable region comprising
CDRH1 consisting of SEQ ID NO: 29,
CDRH2 consisting of SEQ ID NO: 30,
CDRH3 consisting of SEQ ID NO: 31,
and a light chain immunoglobulin variable region comprising
CDRL1 consisting of SEQ ID NO: 32,
CDRL2 consisting of SEQ ID NO: 33,
CDRL3 consisting of SEQ ID NO: 34,
or
(e) a heavy chain immunoglobulin variable region comprising
CDRH1 consisting of SEQ ID NO: 35,
CDRH2 consisting of SEQ ID NO: 36,
CDRH3 consisting of SEQ ID NO: 37,
and a light chain immunoglobulin variable region comprising
CDRL1 consisting of SEQ ID NO: 38,
CDRL2 consisting of SEQ ID NO: 39,
CDRL3 consisting of SEQ ID NO: 40;
wherein the CDRs are as determined according to Kabat.

4. The antibody or antibody fragment of any of claims 1-3, wherein the antibody or antibody fragment is an IgA1, IgA2, IgD, IgE, IgG1, IgG2, IgG3, IgG4, IgM, scFv, IgGΔCH₂, F(ab')2, scFv2CH3, F(ab), scFv4, scFv3, scFv2, dsFv, Fv, scFv-Fc, (scFv)2, diabody, T-body, multispecific antibody, or multivalent antibody.

5. The antibody or antibody fragment of any of claims 1-4, wherein the antibody or antibody fragment is conjugated to another molecule, optionally wherein the antibody or antibody fragment is conjugated to a transmembrane region and an intracellular T-cell receptor (TCR) signaling domain to provide a T-body, optionally wherein the anti-C3d antibody or antibody fragment is conjugated to a label, optionally wherein the anti-C3d antibody or antibody fragment is conjugated to a cytotoxic agent or a therapeutic radioisotope.

6. A pharmaceutical composition comprising the antibody or antibody fragment of any of claims 1-5.

7. A nucleic acid encoding the antibody or antibody fragment of any of claims 1-5, optionally in a vector.

8. A method of preparing an antibody or antibody fragment of any of claims 1-5, the method comprising expressing a nucleic acid encoding the antibody or antibody fragment in a cell.

9. A cell comprising the nucleic acid of claim 7.

10. The anti-C3d antibody or antibody fragment of any of claims 1-5 for treating cancer in a subject, optionally wherein the cancer is **characterized by** surface C3d complement protein, optionally wherein the anti-C3d antibody or antibody fragment is for use with an antibody or antibody fragment to a cell-surface protein other than C3d, and optionally wherein the anti-C3d antibody is a multi-specific antibody that is immunospecific for C3d and a cell-surface protein other than C3d, and contacting the cell with an antibody or antibody fragment to a cell-surface protein other than C3d is accomplished by administering the multi-specific antibody.

11. The anti-C3d antibody or antibody fragment of claim 10, wherein the antibody or antibody fragment to a cell surface protein other than C3d is an anti-CD20 antibody or antibody fragment, optionally wherein the anti-CD20 antibody or antibody fragment is rituximab, ofatumumab, ocrelizumab, veltuzumab, obinutuzumab, PRO131921, or ocaratuzumab, and optionally wherein the cancer cell is a B-cell expressing CD20.

12. The anti-C3d antibody or antibody fragment of claim 10, wherein the antibody or antibody fragment to a cell surface protein other than C3d is an anti-CD33 antibody or antibody fragment, optionally wherein the cancer cell is a myeloid cell.

13. The anti-C3d antibody or antibody fragment of claim 10, wherein the antibody or antibody fragment that specifically binds to a cell surface protein other than C3d is an anti-CD38 antibody or antibody fragment, optionally wherein the cancer cell is a plasma cell or an LKB1-mutant non-small cell lung cancer cell

14. The anti-C3d antibody or antibody fragment of claim 10, wherein the method comprises contacting the cancer cell with an anti-EGFR or anti-ERBB2 antibody or antibody fragment, optionally wherein the cancer cell is from gut, colon, lung, breast, head, neck, pancreas, ovary, endometrium, or brain tissue.
